# EUROPEAN PATENT APPLICATION

(11) **EP 1 321 474 A1**
(43) Date of publication of application: **25.06.2003**
(21) Application number: 01403291.6
(22) Date of filing: 18.12.2001
(51) Int. Cl.: C07K 14/47, A61K 48/00, A61K 38/17, C12N 15/38, C12N 15/11

(54) **A method for inducing apoptosis**

(71) Applicant: UNIVERSITE DE GENEVE, 1211 Genève 4 (CH); INSTITUT NATIONAL DE LA SANTE ET DE LA RECHERCHE MEDICALE (INSERM), 75013 Paris (FR)
(72) Inventor: Irminger, Irmgard, 1207 Genève (CH); Harb, Jean, 44035 Nantes Cedex 01 (FR)
(74) Representative: Jacobson, Claude

(57) **Abstract**

The invention relates to a method for inducing apoptosis of a cell, which method comprises augmenting the activity or amount of BARD1 protein in the cell. In a particular embodiment, this method further comprises augmenting the activity or amount of p53 gene product in the cell. The invention further contemplates inhibiting BRCA activity or expression at the same time.

## Description

The invention relates to a method for inducing apoptosis, by augmenting BARD1 activity in undesired cells.

BARD1 (BRCA1-associated RING domain 1) is described as a nuclear protein that associates with the breast cancer susceptibility gene product BRCA1 (Wu et al., (1996) ; Miki et al., (1994)). The two proteins interact *in vivo* and *in vitro* through their respective RING finger domains (Wu et al., (1996); Meza et al., (1999); Scully et al., (1997)).

As mutations in the BRCA1 RING finger domain (Bienstock et al., (1996)) lead to increased cancer susceptibility, BARD1 was thought to be implicated in BRCA1-mediated tumor suppression. Consistent with this hypothesis, BARD1 mutations were found in breast, ovarian, and uterine tumors (Thai et al., (1998)), and reduced expression of BARD1 is associated with spontaneous breast cancer cases (Yoshikawa et al., (2000)). The *in vitro* repression of BARD1 in murine mammary gland epithelial cells led to a pre-malignant phenotype and genomic instability (Irminger-Finger et al., (1998)). The BARD1/BRCA1 complex was shown to associate with the polyadenylation factor CstF-50 (Kleiman et al., (1999)), presumably to prevent RNA processing at sites of DNA repair (Kleiman and Manley, (2001)). The BRCA1-BARD1 heterodimeric RING finger complex was recently shown to possess ubiquitin ligase activity *in vitro* (Hashizume et al., (2001)).

However, BARD1 and BRCA1 are not consistently co-expressed in different tissues (Irminger-Finger et al., (1998)), suggestive of the existence of independent functions for both genes. Of particular interest in this context is the previously observed high expression level of BARD1 but undetectable BRCA1 concentrations in mouse uterus during the oestrous phase (Irminger-Finger et al., (1998)), i.e. the moment of the oestrous cycle when massive cell death (in part through apoptosis) of the endometrium occurs (Ferenczy, (1993)).

A highly immunogenic cleavage product of BARD1 in apoptotic bodies has also been identified, that could contribute to the antitumoral response mediated by apoptotic bodies (Gautier et al., (2000)).

Yet, the involvement of BARD1 in the regulation of cell death had not been investigated so far.

The invention is based on the identification of BARD1 as a mediator of apoptosis. This function is supported by many consistent data.

First, cell death *in vivo* and *in vitro* is accompanied by increased levels of BARD1 protein and mRNA. Furthermore, overexpression of BARD1 induces cell death with all features of apoptosis. At last, BARD1-repressed cells are defective for the apoptotic response to genotoxic stress.

The proapoptotic activity of BARD1 evidenced by the inventors involves binding to and elevations of p53. BRCA1 is not required for, but partially counteracts, apoptosis induction by BARD1. A tumor-associated mutation Q564H of BARD1 is defective in apoptosis induction, thus supporting a role of BARD1 in tumor suppression by mediating the signaling from proapoptotic stress towards induction of apoptosis.

In the light of these results, the present invention provides a new therapeutic pathway to induce apoptosis in cells.

The inventors more particularly propose to augment the activity or the amount of BARD1 protein in undesired cells.

A subject of the invention is thus a method for inducing apoptosis of a cell, which method comprises augmenting the activity or amount of BARD1 protein in the cell. This is especially helpful to enhance the response to a conventional chemiotherapy in the treatment of cancers.

In a particular embodiment, this method further comprises augmenting the activity or amount of p53 gene product in the cell.

Augmentation of the amount of BARD1 protein in the cell may be achieved by transfecting the cell with a BARD1 expressing vector, *e.g.* in the form of naked DNA, or as viral vector. Such a vector preferably encodes a BARD1 protein comprising an amino acid sequence shown as SEQ ID NO:2.

Cell specific killing may be performed by using appropriate promoters, *e.g.* telomerase promoter for tumor cell killing.

The BARD1 encoding nucleic acid may be associated with a p53 expressing nucleic acid for the manufacture of a therapeutic association useful for inducing apoptosis of undesired cells. Cells may more particularly be transfected with a p53 expressing vector, or the vector that encodes the BARD1 protein may be designed to further encode a p53 gene product. This is especially advantageous in p53 deficient cells.

Another subject of the invention is an expression vector that comprises
i) a BARD1 encoding nucleic acid ; and
ii) a p53 encoding nucleic acid,
said nucleic acids being operatively associated with expression control sequences.

Another subject of the invention is a pharmaceutical composition comprising the above expression vector, in association with a pharmaceutically acceptable carrier.

The invention also provides a pharmaceutical composition comprising :
- an expression vector that comprises a BARD1 encoding nucleic acid operatively associated with expression control sequences ; and
- an expression vector that comprises a p53 encoding nucleic acid operatively associated with expression control sequences ; in association with a pharmaceutically acceptable carrier.

A further subject of the invention is a kit comprising :
- in a first container, a pharmaceutical composition comprising an expression vector that comprises a BARD1 encoding nucleic acid operatively associated with expression control sequences ; and
- in a second container, a pharmaceutical composition comprising an expression vector that comprises a p53 encoding nucleic acid operatively associated with expression control sequences.

BARD1 encoding nucleic acid may also be associated with a p53 gene product or an agent that stimulates p53 expression or activity for the manufacture of a therapeutic association useful for inducing apoptosis of undesired cells.

Augmentation of the amount of BARD1 protein in the cell may also be achieved by administering the cell with a BARD1 protein, or a BARD1 peptide that possesses the p53 stabilizing, hence apoptotic activity.

Another embodiment consists in stimulating BARD1 endogenous expression or activity in the cell. Upregulation of BARD1 protein in the cell could more particularly be achieved by transfer of stimulatory agents, such as small peptides that stabilize BARD1.

The present invention further provides a method for inducing apoptosis in a cell, wherein one inhibits BRCA1 activity or expression while stimulating BARD1 activity or expression.

For that purpose, any agent that inhibits BRCA1 can be used, for example in association with a BARD1 encoding nucleic acid, or a BARD1 protein.

A particular subject of the invention is thus a pharmaceutical composition comprising :
i) a BARD1 encoding nucleic acid, and
ii) a BRCA1 antisense nucleic acid ;
in association with a pharmaceutically acceptable carrier.

Another subject of the invention is a kit comprising
- in a first container, a pharmaceutical composition comprising an expression vector that comprises a BARD1 encoding nucleic acid operatively associated with expression control sequences ; and
- in a second container, a pharmaceutical composition comprising a BRCA1 antisense nucleic acid.

The invention is described in greater detail below.

### General definitions

In accordance with the present invention there may be employed conventional molecular biology, microbiology, and recombinant DNA techniques within the skill of the art. Such techniques are explained fully in the literature. See, *e.g.*, Sambrook et al., (1989) ; B.D. Hames & S.J. Higgins, (1984); R.I. Freshney, (1986); Perbal, (1984); F.M. Ausubel *et al.* (1994).

The nucleic acid used in the context of the present invention can be a deoxyribonucleic acid (DNA) or a ribonucleic acid (RNA). Among DNAs, possible alternatives include a complementary DNA (cDNA), a genomic DNA (gDNA), a hybrid sequence or a synthetic or semi-synthetic sequence. A further possibility is a nucleic acid modified chemically, for example, for the purpose of increasing its resistance to nucleases, its cell penetration or cell targeting, its therapeutic efficacy, and the like. These nucleic acids can be of human, animal, plant, bacterial, viral, synthetic and the like, origin. They may be obtained by any technique known to a person skilled in the art, and in particular by screening of libraries, by chemical synthesis or alternatively by mixed methods including the chemical or enzymatic modification of sequences obtained by screening of libraries. As mentioned later, they can, moreover, be incorporated in vectors such as plasmid, viral or chemical vectors.

A "*coding sequence"* or a sequence *"encoding"* an expression product, such as a RNA, polypeptide, protein, or enzyme, is a nucleotide sequence that, when expressed, results in the production of that RNA, polypeptide, protein, or enzyme, *i.e.*, the nucleotide sequence encodes an amino acid sequence for that polypeptide, protein or enzyme. A coding sequence for a protein may include a start codon (usually ATG) and a stop codon.

The term *"gene",* also called a *"structural gene"* means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription.

A coding sequence is *"under the control of* or *"operatively associated with"* expression (i.e. transcription and/or translation) control sequences in a cell when RNA polymerase transcribes the coding sequence into RNA, particularly mRNA, which is then trans-RNA spliced (if it contains introns) and translated into the protein encoded by the coding sequence.

A *"promoter"* or *"promoter sequence"* is a DNA regulatory region capable of binding RNA polymerase in a cell and initiating transcription of a downstream (3' direction) coding sequence. The promoter sequence is generally bounded at its 3' terminus by the transcription initiation site and extends upstream (5' direction) to include the minimum number of bases or elements necessary to initiate transcription at levels detectable above background. Within the promoter sequence are found a transcription initiation site (conveniently defined for example, by mapping with nuclease SI), as well as protein binding domains (consensus sequences) responsible for the binding of RNA polymerase. The promoter may be operatively associated with other expression control sequences, including enhancer and repressor sequences.

Promoters which may be used to control gene expression include, but are not limited to, cytomegalovirus (CMV) promoter (V.S. Patents No. 5,385,839 and No. 5,168,062), the SV40 early promoter region (Benoist et al. (1981), the promoter contained in the 3' long terminal repeat of Rous sarcoma virus (Yamamoto et al. (1980)), the herpes thymidine kinase promoter (Wagner et al. (1981)), the regulatory sequences of the metallothionein gene (Brinster et al. (1982)) ; prokaryotic expression vectors such as the beta-lactamase promoter (Villa-Komaroff et al. (1978)), or the *tac* promoter (DeBoer et al. (1983) and (1980) "Useful proteins from recombinant bacteria" in Scientific American) ; promoter elements from yeast or other fungi such as the Gal 4 promoter, the ADC (alcohol dehydrogenase) promoter, PGK (phosphoglycerol kinase) promoter, alkaline phosphatase promoter; and transcriptional control regions that exhibit hematopoietic tissue specificity, in particular: beta-globin gene control region which is active in myeloid cells (Mogram et al. (1985) ; Kollias et al. (1986)), hematopoietic stem cell differentiation factor promoters, erythropoietin receptor promoter (Maouche et al. (1991)), etc.

The terms "*mutant*" and *"mutation"* mean any detectable change in genetic material, *e.g.* DNA, or any process, mechanism, or result of such a change. This includes gene mutations, in which the structure *(e.g.* DNA sequence) of a gene is altered, any gene or DNA arising from any mutation process, and any expression product *(e.g.* protein or enzyme) expressed by a modified gene or DNA sequence. The term "variant" may also be used to indicate a modified or altered gene, DNA sequence, enzyme, cell, etc., *i.e.*, any kind of mutant.

*"Sequence-conservative variants"* of a polynucleotide sequence are those in which a change of one or more nucleotides in a given codon position results in no alteration in the amino acid encoded at that position.

*"Function-conservative variants"* are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Such changes are expected to have little or no effect on the apparent molecular weight or isoelectric point of the protein or polypeptide. Amino acids other than those indicated as conserved may differ in a protein or enzyme so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide or enzyme which has at least 60 % amino acid identity as determined by BLAST or FASTA algorithms, preferably at least 75 %, most preferably at least 85%, and even more preferably at least 90 %, and which has the same or substantially similar properties or functions as the native or parent protein or enzyme to which it is compared.

As used herein, the term *"homologous"* in all its grammatical forms and spelling variations refers to the relationship between proteins that possess a "common evolutionary origin," including proteins from superfamilies *(e.g.,* the immunoglobulin superfamily) and homologous proteins from different species *(e.g.,* myosin light chain, etc.) (Reeck *et al.,* (1987)). Such proteins (and their encoding genes) have sequence homology, as reflected by their sequence similarity, whether in terms of percent similarity or the presence of specific residues or motifs at conserved positions.

Accordingly, the term "sequence *similarity*" in all its grammatical forms refers to the degree of identity or correspondence between nucleic acid or amino acid sequences of proteins that may or may not share a common evolutionary origin *(see* Reeck *et al., supra).* However, in common usage and in the instant application, the term "homologous," when modified with an adverb such as "highly," may refer to sequence similarity and may or may not relate to a common evolutionary origin.

In a specific embodiment, two DNA sequences are "substantially homologous" or "substantially similar" when at least about 80 %, and most preferably at least about 90 or 95 %, of the nucleotides match over the defined length of the DNA sequences, as determined by sequence comparison algorithms, such as BLAST, FASTA, DNA Strider, etc. An example of such a sequence is an allelic or species variant of the specific genes of the invention. Sequences that are substantially homologous can be identified by comparing the sequences using standard software available in sequence data banks, or in a Southern hybridization experiment under, for example, stringent conditions as defined for that particular system.

Similarly, in a particular embodiment, two amino acid sequences are *"substantially homologous"* or *"substantially similar'* when greater than 80 % of the amino acids are identical, or greater than about 90 % are similar (functionally identical). Preferably, the similar or homologous sequences are identified by alignment using, for example, the GCG (Genetics Computer Group, Program Manual for the GCG Package, *Version 7,* Madison, Wisconsin) pileup program, or any of the programs described above (BLAST, FASTA, etc.).

A nucleic acid molecule is *"hybridizable"* to another nucleic acid molecule, such as a cDNA, genomic DNA, or RNA, when a single stranded form of the nucleic acid molecule can anneal to the other nucleic acid molecule under the appropriate conditions of temperature and solution ionic strength *(see* Sambrook *et al., supra).* The conditions of temperature and ionic strength determine the "stringency" of the hybridization. For preliminary screening for homologous nucleic acids, low stringency hybridization conditions, corresponding to a Tm (melting temperature) of 55°C, can be used, *e.g.*, 5x SSC, 0.1 % SDS, 0.25 % milk, and no formamide ; or 30 % formamide, 5x SSC, 0.5 % SDS). Moderate stringency hybridization conditions correspond to a higher Tm, *e.g.*, 40 % formamide, with 5x or 6x SCC. High stringency hybridization conditions correspond to the highest Tm, *e.g.*, 50 % formamide, 5x or 6x SCC. SCC is a 0.15 M NaCI, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived *(see* Sambrook *et al., supra,* 9.50-9.51). For hybridization with shorter nucleic acids, i.e., oligonucleotides, the position of mismatches becomes more important, and the length of the oligonucleotide determines its specificity *(see* Sambrook *et al., supra,* 11.7-11.8). A minimum length for a hybridizable nucleic acid is at least about 10 nucleotides ; preferably at least about 15 nucleotides ; and more preferably the length is at least about 20 nucleotides.

In a specific embodiment, the term *"standard hybridization conditions"* refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

The terms *"vector", "cloning vector"* and *"expression vector"* mean the vehicle by which a DNA or RNA sequence (*e.g.* a foreign gene) can be introduced into a host cell, so as to transform the host and promote expression (e.g. transcription and translation) of the introduced sequence. Vectors include plasmids, phages, viroses, etc.; they are discussed in greater detail below.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. A "cassette" refers to a DNA coding sequence or segment of DNA that codes for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct." A common type of vector is a "plasmid", which generally is a self-contained molecule of doublestranded DNA, usually of bacterial origin, that can readily accept additional (foreign) DNA and which can readily introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts. Non-limiting examples include pKK plasmids (Clonetech), pUC plasmids, pET plasmids (Novagen, Inc., Madison, WI), pRSET or pREP plasmids (Invitrogen, San Diego, CA), or pMAL plasmids (New England Biolabs, Beverly, MA), and many appropriate host cells, using methods disclosed or cited herein or otherwise known to those skilled in the relevant art. Recombinant cloning vectors will often include one or more replication systems for cloning or expression, one or more markers for selection in the host, *e.g.* antibiotic resistance, and one or more expression cassettes.

The terms *"express"* and *"expression"* mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, *e.g.* the resulting protein, may also be said to be "expressed" by the cell. An expression product can be characterized as intracellular, extracellular or secreted. The term *"intracellular"* means something that is inside a cell. The term *"extracellular'* means something that is outside a cell. A substance is "secreted" by a cell if it appears in significant measure outside the cell, from somewhere on or inside the cell.

The term *"transfection"* means the introduction of a foreign nucleic acid into a cell. The term *"transformation"* means the introduction of a "foreign" *(i.e.* extrinsic or extracellular) gene, DNA or RNA sequence to a host cell, so that the host cell will express the introduced gene or sequence to produce a desired substance, typically a protein or enzyme coded by the introduced gene or sequence. The introduced gene or sequence may also be called a "cloned" or "foreign" gene or sequence, may include regulatory or control sequences, such as start, stop, promoter, signal, secretion, or other sequences used by a cell's genetic machinery. The gene or sequence may include nonfunctional sequences or sequences with no known function. A host cell that receives and expresses introduced DNA or RNA bas been "transformed" and is a "transformant" or a "clone." The DNA or RNA introduced to a host cell can come from any source, including cells of the same genus or species as the host cell, or cells of a different genus or species.

The term *"host cell*" means any cell of any organism that is selected, modified, transformed, grown, or used or manipulated in any way, for the production of a substance by the cell, for example the expression by the cell of a gene, a DNA or RNA sequence, a protein or an enzyme. Host cells can further be used for screening or other assays, as described *infra.*

The term *"expression system"* means a host cell and compatible vector under suitable conditions, *e.g.* for the expression of a protein coded for by foreign DNA carried by the vector and introduced to the host cell. Common expression systems include *E. coli* host cells and plasmid vectors, insect host cells and *Baculovirus* vectors, and mammalian host cells and vectors. In a specific embodiment, the protein of interest is expressed in COS-1 or C₂C₁₂ cells. Other suitable cells include CHO cells, HeLa cells, 293T (human kidney cells), mouse primary myoblasts, and NIH 3T3 cells.

"*Pharmaceutically*" or *"pharmaceutically acceptable"* refer to molecular entities and compositions that do not produce an adverse, allergic or other untoward reaction when administered to an animal, or a human, as appropriate.

As used herein, "*pharmaceutically acceptable carrier"* includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

*"Apoptosis"* as described herein is a form of cell death (programmed cell death) that exhibits stereotypic morphological changes as reviewed in Raff (1998). The most striking feature is breakage of chromatin DNA into nucleosome units Apoptosis can be measured, e.g., by the propidium iodide flow cytometry assay described in Dengler et al. (1995), or by the in situ terminal deoxynucleotidyl transferase and nick translation assay (TUNEL analysis) described in Gorczyca, (1993).

### BARD1

"*BARD1 protein"* refers to human BARD1 protein, *e.g.* comprising the aminoacid sequence set forth as SEQ ID n° 2, or homologous proteins, including BARD1 protein of other species and function-conservative variants, as defined above.

Similarly, the term "*BARD1 gene"* or "*BARD1 nucleic acid"* refers to a gene or nucleic acid that encodes a BARD1 protein. The cDNA that encodes human BARD1 protein is shown as SEQ ID n° 1. However, homologous sequences, in particular highly homologous sequences that are highly hybridizable to SEQ ID n° 1 or its complementary sequence, are also encompassed.

In the context of the present invention, *"augmenting the activity or amount of BARD1 protein"* means either introducing an exogenous BARD1 protein (either in the protein form, or in the form of a BARD1 encoding nucleic acid), or stimulating the endogenous BARD1 expression or activity. Stimulating the endogenous BARD1 activity more particularly encompasses enhancing the stability of the protein. The desired amount of BARD1 to be obtained is at least as high as the constitutive BARD1 amount in control cells.

The purpose of the invention is to augment or enhance the level of BARD1 activity to trigger apoptosis of the cells. Preferably, the level of BARD1 activity is enhanced to be at least 10 % higher than the constitutive control level, preferably at least 20 %, most preferably at least 50 %.

The term "*BARD1 activity"* refers to the biological functions of BARD1, including a p53 stabilizing activity, as well as BRCA1 dependent and independent functions, *e.g.* its ability to interact with various factors (CstF-50, NF-κB, Bcl-3). BARD1 apoptotic activity is sustained by a BRCT repeat (aminoacids 592 to 765) containing C-terminal domain of the BARD1 protein.

The term *"BARD1 peptide"* refers to a peptide fragment derived from the BARD1 protein as above defined, and that preferably exhibits a p53 stability hence apoptotic activity, similar as the whole BARD1 protein. Such BARD1 peptides preferably comprise the BRCT domain and/or the upstream adjacent region of BARD1.

### p53

In a particular embodiment, the present invention further contemplates augmenting the activity or amount of p53 gene product in the cell, in addition to the BARD1 augmentation.

The gene for the nuclear phosphoprotein p53 is the most commonly mutated gene yet identified in human cancers (Vogelstein (1990)). Missense mutations occur in tumors of the colon, lung, breast, ovary, bladder, and several other organs.

In the context of the present invention, "*p53 gene"* and *"p53 protein or gene product"* refer to the human form of the gene or gene product, or homologous forms, including p53 of other species or function-conservative variants.

As described above for the augmentation of BARD1, *"augmenting the activity or amount of p53 protein"* means either introducing an exogenous p53 protein (either in the protein form, or in the form of a p53 coding nucleic acid), or stimulating the endogenous p53 expression or activity. Stimulating the endogenous p53 activity more particularly encompasses enhancing the stability of the protein, *e.g.* inhibiting p53 rapid turnover. The desired amount of p53 to be obtained is at least as high as the constitutive p53 amount in control cells.

The purpose of the invention is to augment or enhance the level of p53 activity to trigger apoptosis of the cells. Preferably, the level of p53 is enhanced to be at least 10 % higher than the constitutive control level, preferably at least 20 %, most preferably at least 50 %.

The term *"p53 activity*" refers to the biological functions of p53, including p53 functions in G1 arrest and in apoptosis induction, as well as transcriptional activation of the respective target genes.

### Association of BARD1 and p53 therapies

In a preferred embodiment, the present invention contemplates augmenting or stimulating BARD1, and augmenting or stimulating p53 in a combined action. This double action is intended to enhance the BARD1 apoptotic effect, since this effect occurs through p53 pathway. Although p53 is mutated of deleted in more than 50 % of all cancers, tumors exist that are resistant to apoptosis inducing treatments such as chemotherapy and radiation. The inventors propose that BARD1 deficiency could be the reason for such resistance. Combined treatments, aimed at induction of p53 and BARD1 therefore represent straight forward strategies. Evidence exists supporting mutual stabilization of BARD1 and p53. Therefore a combined treatment using both genes or proteins is advantageous.

In the context of the present invention, a therapeutic composition comprising a BARD1 encoding nucleic acid, a BARD1 protein, or an agent that stimulates BARD1 expression or activity may be combined with a therapeutic composition comprising a p53 encoding nucleic acid, a p53 protein or gene product, or an agent that stimulates p53 expression or activity. Any combination is encompassed. The composition may be part of the same pharmaceutical formulation (in this case, though, one skilled in the art would naturally favour an association of the same type of products, *i.e*. a BARD1 nucleic acid with a p53 nucleic acid, or a BARD1 protein with a p53 protein, or an agent that stimulates BARD1 expression or activity with an agent that stimulates p53 expression or activity). This is particularly adapted for the co-transfection of cells with a BARD1 nucleic acid and a p53 nucleic acid. In that event the cells may be transfected with the two types of vectors, or the vector that encodes the BARD1 protein may be designed to further encode a p53 gene product.

Alternatively, the compositions may be formulated in separated pharmaceutical products, that can be simultaneously or sequentially administered. Kits comprising two recipients, wherein a first container contains a pharmaceutical composition comprising a BARD1 encoding nucleic acid, a BARD1 protein, or an agent that stimulates BARD1 expression or activity, and a second container contains a pharmaceutical composition of a p53 encoding nucleic acid, a p53 protein or gene product, or an agent that stimulates p53 expression or activity, are part of the present invention.

All the above embodiments are referred herein as a *"therapeutic association".*

### BRCA1/BARD1 ratio:

The BRCA1 gene has been cloned in 1994 (Miki et al.). It encodes an 1863 aminoacids protein with an apparent molecular mass of 220 kDa (Chen et al., (1995) ; Scully et al., (1996)). Frequent loss of the wild-type allele in tumors of mutation carriers suggests that BRCA1 acts as a tumor suppressor gene. Several lines of evidence indicate that BRCA1 would have a role in DNA damage-dependent replication checkpoint response (Scully et al., (1997); Zhong et al. (1999); Wang et al (2000)). Accordingly, its ability to bind directly DNA structures may be important for its role in DNA repair (Paull et al., (2001)). BRCA1 would have a role in transcription-coupled repair of oxidative damages (Gowen et al., (1998); Abbott et al., (1999)).

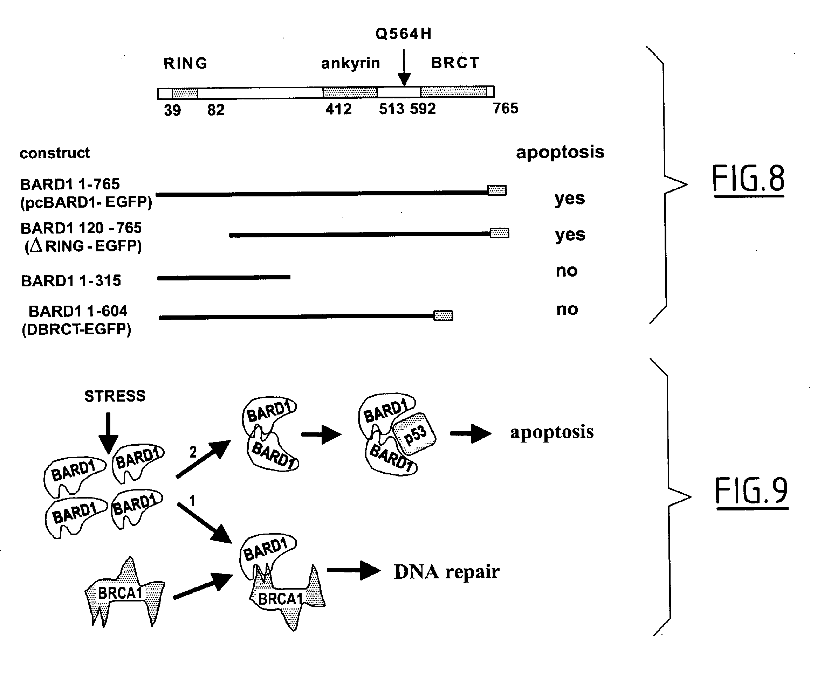

Based on the BRCA1-dependent BARD1 functions described previously and on the BRCA1-indepedent BARD1 function described in this paper, we propose a "dual mode of action" model of BARD1 function (Fig. 9). In the survival mode, BARD1 acts in a heterodimeric complex with BRCA1 and is involved in DNA repair and cell survival. In the death mode, BARD1 acts independently of BRCA1, and induces apoptosis. Thus, according to this model, the ratio of BRCA1 and BARD1 expressed in a given cell would determine cell fate; a high BRCA1/BARD1 ratio would lead to DNA repair and survival, while a low ratio would lead to cell death.

In the context of the present invention, it is therefore advantageous to inhibit BRCA1 activity or expression while stimulatting BARD1 activity or expression.

This can be achieved by various methods that one skilled in the art is familiar with.

In a particular embodiment cells can be cotransfected with a BRCA1 antisense nucleic acid and a BARD1 encoding nucleic acid. Such cotransfection can be preferably accomplished by using two types of vectors (one transferring BRCA11 antisense sequence, the other transferring BARD1 encoding sequence, operately associated with expression control sequences).

An *"antisense nucleic acid"* is a single stranded nucleic acid molecule which, on hybridizing under cytoplasmic conditions with complementary bases in an RNA or DNA molecule, inhibits the latter's role. If the RNA is a messenger RNA transcript, the antisense nucleic acid is a countertranscript or mRNA-interfering complementary nucleic acid. As presently used, "antisense" broadly includes RNA-RNA interactions, RNA-DNA interactions, ribozymes and RNase-H mediated arrest. Antisense nucleic acid molecules can be encoded by a recombinant gene for expression in a cell *(e.g.,* U.S. Patent No. 5,814,500 ; U.S. Patent No. 5,811,234), or alternatively they can be prepared synthetically *(e.g.,* U.S. Patent No. 5,780,607).

Inhibition of BRCA1 can also be achieved by sing inhibitory antibodies, for example.

Agents that inhibit BRCA1 endogenous expression can be useful too.

### Undesired cells

In one embodiment, cells, the apoptosis of which is desired, are cells infected with a pathogenic agent, such as a virus or a bacteria.

In another embodiment, the present invention is advantageously used for the destruction of hyperproliferating (i.e. abnormally proliferating) cells. It is thus applicable to the destruction of tumour cells or of smooth muscle cells of the vascular wall (restenosis).

Preferably, the present invention addresses treatment of cancers wherein mutated forms of p53 are observed, such as, in particular colorectal cancer, breast, ovarian and uterine cancer, lung cancer, stomach cancer, cancer of the oesophagus, B-cell lymphomas, bladder cancer, prostate, and the like, but also brain tumors such as astrocytes derived tumors.

The undesired cells targeted by the present invention may be cells of human patients, or of other animal species, preferably mammals, including rodents, cattle, sheep, feline and canine for example.

### Gene therapy

In accordance with the present invention, undesired cells may be transfected with a BARD1 nucleic acid.

In a particular embodiment, the cell is co-transfected with a p53 nucleic acid. This p53 coding sequence may be administered simultaneously, or separately with regard to the BARD1 transfection. A transfection may be advantageously achieved by using a vector that encodes the BARD1 protein and may be designed to further encode a p53 gene product.

Preferably, the nucleic acid according to the invention forms part of a vector. Such vector is a nucleic acid comprising a BARD1 coding sequence operatively associated with sequences that control expression of BARD1 in a cell transfected with the vector.

The use of such a vector indeed makes it possible to improve the administration of the nucleic acid into the cells to be treated, and also to increase its stability in the said cells, which makes it possible to obtain a durable therapeutic effect. Furthermore, it is possible to introduce several nucleic acid sequences into the same vector, which also increases the efficacy of the treatment.

The vector used may be of diverse origin, as long as it is capable of transforming animal cells, preferably human tumour cells. In a preferred embodiment of the invention, a viral vector is used which can be chosen from adenoviruses, retroviruses, adeno-associated viruses (AAV), herpes virus, cytomegalovirus (CMV), vaccinia virus and the like. Vectors derived from adenoviruses, retroviruses or AAVs, HIV-derived retroviral vectors, incorporating heterologous nucleic acid sequences have been described in the literature (Akli et al., (1993); Stratford-Perricaudet et al. (1990) ; EP 185 573, Levrero et al. (1991) ; Le Gal la Salle et al. (1993) ; Roemer et al. (1992) ; Dobson et al. (1990) ; Chiocca et al. (1990) ; Miyanohara et al. (1992) ; WO 91/18088).

The present invention therefore also relates to any recombinant virus comprising, inserted into its genome, a BARD1 nucleic acid sequence as defined before, optionally accompanied with a p53 nucleic acid sequence.

Advantageously, the recombinant virus according to the invention is a defective virus. The term *"defective virus"* designates a virus incapable of replicating in the target cell. Generally, the genome of the defective viruses used within the framework of the present invention is therefore devoid of at least the sequences necessary for the replication of the said virus in the infected cell. These regions can either be removed (completely or partially), or rendered non-functional, or substituted by other sequences and especially by the BARD1 nucleic acid of the invention. Preferably, the defective virus nevertheless conserves the sequences of its genome which are necessary for the encapsulation of the viral particles.

It is particularly advantageous to use the nucleic acid sequences of the invention in a form incorporated in an adenovirus, an AAV or a defective recombinant retrovirus.

As regards adenoviruses, various serotypes exist whose structure and properties vary somewhat, but which are not pathogenic for man, and especially non-immunosuppressed individuals. Moreover, these viruses do not integrate into the genome of the cells which they infect, and can incorporate large fragments of exogenous DNA. Among the various serotypes, the use of the type 2 or 5 adenoviruses (Ad2 or Ad5) is preferred within the framework of the present invention. In the case of the Ad5 adenoviruses, the sequences necessary for the replication are the E1A and E1 B regions.

The defective recombinant viruses of the invention can be prepared by homologous recombination between a defective virus and a plasmid carrying, *inter alia,* the BARD1 encoding sequence (Levrero et al. (1991); Graham, (1984)). The homologous recombination is produced after co-transfection of the said viruses and plasmid into an appropriate cell line. The cell line used should preferably (i) be transformable by the said elements, and (ii), contain sequences capable of complementing the part of the genome of the defective virus, preferably in integrated form so as to avoid the risks of recombination. As example of a line which can be used for the preparation of defective recombinant adenoviruses, there may be mentioned the human embryonic kidney line 293 (Graham et al. (1977)) which contains especially, integrated into its genome, the left part of the genome of an Ad5 adenovirus (12%). As example of a line which can be used for the preparation of defective recombinant retroviruses, there may be mentioned the CRIP line (Danos et al. (1988)). Alternative vectors, such as shuttle vectors, can also be used that permit the cloning of the desired gene in the vector backbone.

Then the viruses which have multiplied are recovered and purified according to conventional molecular biology techniques.

Targeted gene delivery is described in International Pat. Publication WO 95/28494, published October 1995.

Alternatively, the vector can be introduced *in vivo* by lipofection. For the past decade, there has been increasing use of liposomes for encapsulation and transfection of nucleic acids *in vitro.* Information regarding liposome is provided in the "pharmaceutical composition" section of the present application as well. Synthetic cationic lipids designed to limit the difficulties and dangers encountered with liposome mediated transfection can be used to prepare liposomes for *in vivo* transfection of a gene encoding a marker (Felgner, et. al. (1987) ; Mackey, et al., (1988). The use of cationic lipids may promote encapsulation of negatively charged nucleic acids, and also promote fusion with negatively charged cell membranes (Felgner et al. (1989). The use of lipofection to introduce exogenous genes into the specific organs *in vivo* has certain practical advantages. Molecular targeting of liposomes to specific cells represents one area of benefit. It is clear that directing transfection to particular cell types would be particularly advantageous in a tissue with cellular heterogeneity, such as pancreas, liver, kidney, and the brain. Lipids may be chemically coupled to other molecules for the purpose of targeting (see Mackey, et. al., supra). Targeted peptides, *e.g.* hormones or neurotransmitters, and proteins such as antibodies, or non-peptide molecules could be coupled to liposomes chemically.

It is also possible to introduce the vector *in vivo* as a naked DNA plasmid. Naked DNA vectors for gene therapy can be introduced into the desired host cells by methods known in the art, e.g., transfection, electroporation, microinjection, transduction, cell fusion, DEAE dextran, calcium phosphate precipitation, use of a gene gun, or use of a DNA vector transporter (see, e.g., Wu et al., (1992); Wu et al. (1988)).

In a particular embodiment, the invention provides an expression vector that comprises
i) a BARD1 encoding nucleic acid ; and
ii) a p53 encoding nucleic acid,
said nucleic acids being operatively associated with expression control sequences.

BARD1 encoding sequence and p53 encoding sequence are either controlled together by the same regulatory sequences or each of BARD1 and p53 is under the control of their proper regulatory sequences that can be identical or different from one another.

### Peptide therapy

In another embodiment, the present invention provides a method for inducing apoptosis of a cell, which method comprises augmenting the amount of BARD1 protein in the cell by administering the cell with a BARD1 protein, or a BARD1 peptide.

For that purpose, the BARD1 protein or peptide may be chemically or enzymatically modified to improve stability or biovailability.

In a particular embodiment, a p53 gene product may be simultaneously or separately administered in a therapeutic association.

### Stimulating agents

In another embodiment, augmenting the amount or activity of BARD1 protein in a cell is achieved by stimulating BARD1 endogenous expression or activity in the cell.

Such stimulating agents may be identified by a screening method. This screening method preferably comprises contacting an agent to be tested with a BARD1 expressing cell, and assessing the level of expression or activity of BARD1 in the cell.

Agents, such as small molecules, that stabilize BARD1 (*e.g.* by enhancing the interaction between BRCA1 and BARD1, Joukov et al (2001)) are encompassed.

The enhancement of level of expression or activity of BARD1 in comparison with a BARD1 expressing cell that was not subjected to this agent, is indicative of an agent that shows a stimulating property toward BARD1.

Test agents can be of any type, including natural or synthetic compounds or mixtures of compounds.

The component may be structurally defined or of unknown structure, *e.g.* in the form of a biological extract.

While stimulating BARD1 expression or activity, it may be useful to stimulate p53 expression or activity as well, or to augment the p53 amount, by any means as described above. Agents that inhibit the rapid turnover of p53, are particularly useful.

### Pharmaceutical compositions

The present invention further encompasses preparing pharmaceutical compositions useful for the above therapeutic methods. Examples of pharmaceutical formulations are provided hereafter. They can be readily adapted to the preparation of any type of pharmaceutical composition, comprising an active ingredient selected from BARD1 (and optionally p53) encoding nucleic acid, BARD1 (and optionally p53) proteins, and BARD1 (and optionally p53) stimulating agents.

A preferred subject of the present invention is a pharmaceutical composition containing at least a BARD1 nucleotide sequence as defined above, preferably inserted in a recombinant virus.

The pharmaceutical compositions of the invention can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected, optionally directly into the tumour to be treated. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses of nucleic acids (sequence or vector) used for the administration can be adapted as a function of various parameters, and in particular as a function of the mode of administration used, of the relevant pathology, of the nucleic acid to be expressed, or alternatively of the desired duration of treatment. Generally, with regard to recombinant viruses, the latter are formulated and administered in the form of doses of between 10⁴ and 10¹⁴ pfu/ml, and preferably 10⁶ to 10¹⁰ pfu/ml. The term pfu ("plaque forming unit") corresponds to the infectivity of a virus solution, and is determined by infecting an appropriate cell culture and measuring, generally after 48 hours, the number of plaques of infected cells. The techniques for determining the pfu titer of a viral solution are well documented in the literature.

To prepare pharmaceutical compositions for peptide therapy, an effective amount of the BARD1 protein may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

A BARD1 protein can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In terms of using peptide therapeutics as active ingredients, U.S. patents 4,608,251 ; 4,601,903 ; 4,599,231 ; 4,599,230 ; 4,596,792 and 4,572,770 provide useful information.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCI solution and either added to 1000 ml of hypodermoclysis fluid or injected at the proposed site of infusion, (see for example, "Remington's Pharmaceutical Sciences" 15^{th} Edition, pages 1035-1038 and 1570-1580). Some variation in dosage will necessarily occur depending on the condition of the subject being treated. The person responsible for administration will, in any event, determine the appropriate dose for the individual subject.

The active BARD1 protein may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, *e.g.* tablets or other solids for oral administration ; liposomal formulations ; time release capsules ; and any other form currently used, including cremes.

Other routes of administration are contemplated, including nasal solutions or sprays, aerosols or inhalants, or vaginal or rectal suppositories and pessaries.

In certain embodiments, the use of liposomes and/or nanoparticles is contemplated for the introduction of BARD1 protein into host cells. The formation and use of liposomes is generally known to those of skill in the art, and is also described below, as well as in the *"gene therapy"* section.

Nanocapsules can generally entrap compounds in a stable and reproducible way. To avoid side effects due to intracellular polmeric overloading, such ultrafine particles (sized around 0.1 µm) should be designed using polymers able to be degraded *in vivo.* Biodegradable polyalkylcyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core.

The following information may also be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state, to a loosely packed, less-ordered structure, known as the fluid state. This occurs as a characteristic phase-transition temperature and results in an increase in permeability to ions, sugars and drugs.

Liposomes interact with cells via four different mechanisms : endocytosis by phagocytic cells of the reticuloendothelial system such as macrophages and neutrophils ; adsorption to the cell surface, either by non-specific weak hydrophobic or electrostatic forces, or by specific interactions with cell-surface components ; fusion with the plasma cell membrane by insertion of the lipid bilayer of the liposome into the plasma membrane, with simultaneous release of liposomal contents into the cytoplasm ; and by transfer of liposomal lipids to cellular or subcellular membrane, or *vice versa,* without any association of the liposome contents. Varying the liposome formulation can alter which mechanism is operative, although more than one may operate at the same time.

The below figures and examples illustrate the invention without limiting its scope.

### DESCRIPTION OF THE FIGURES

**Figure 1.** BARD1 expression is upregulated upon stress.

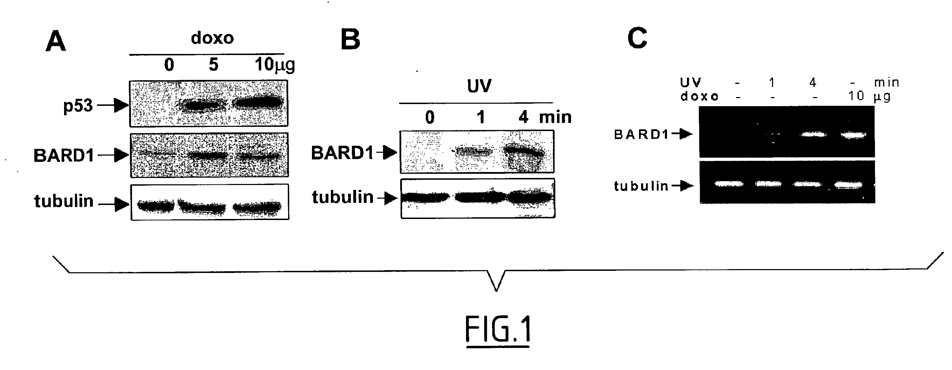

   Figure 1A : BARD1 protein and mRNA is upregulated in cells exposed to genotoxic stress. Increase of p53 and BARD1 protein in TAC-2 cells, treated with doxorubicin (0, 5, or 10 µg/ml) for 12 hours, is demonstrated by Western blotting. Figure 1B : BARD1 expression is increased after exposure to UV light for 1 to 4 min (2.5 to 10 Jm⁻²). The expression of BARD1 was tested with anti-Bard1 antibody (PVC, Irminger-Finger et al., 1998), and p53 expression was probed with anti-p53 antibody (FL-393, Santa Cruz). Figure 1C : RT-PCR of RNA from mouse ES cells exposed to UV light for 0, 1 or 4 min (0, 2.5, or 10 Jm⁻²) or treated with doxorubicin (10 µg/ml) shows an increase of BARD1 expression, while expression of the control house keeping gene β-tubulin remains constant.
**Figure 2.** BARD1 overexpression induces apoptosis *in vitro.*
   TAC-2 cells were transfected with pcDNA3 vector sequences, BARD1 or the p53 sequences cloned into pcDNA3. Apoptosis induction was monitored by TUNEL staining 24 hours after transfection. Bar, 10 µm. Ten photographic fields were randomly chosen to evaluate the mean percentage of stained nuclei (Bar chart).

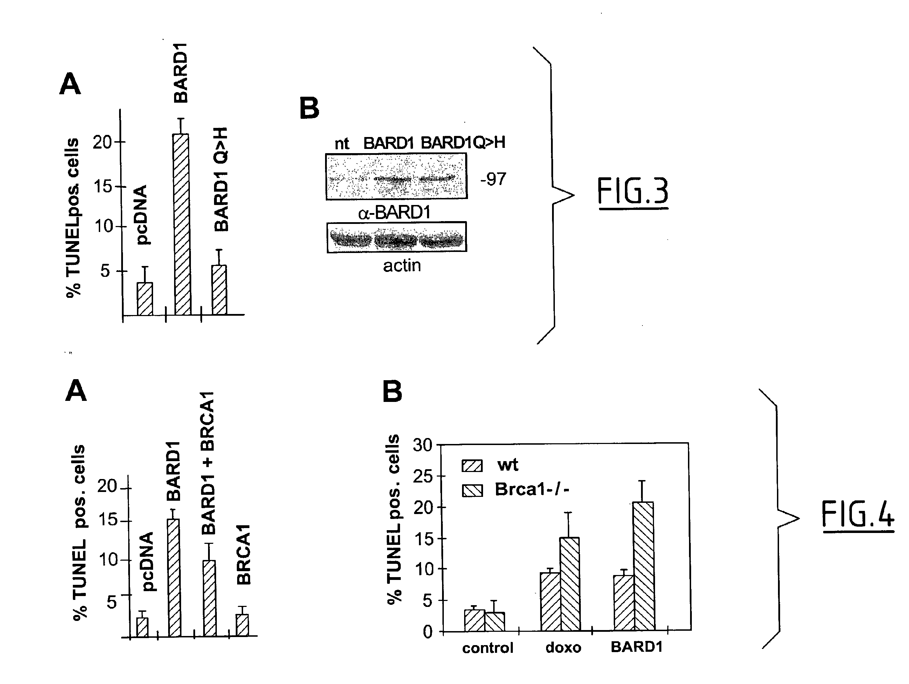

Figure 3. Mapping of proapoptotic activity.
   BARD1 wild type or mutant protein, were expressed by transient transfection in mouse ES cells.
   Figure 3A : Apoptosis induction in control transfectants and transfected cells was monitored 24 hours after transfection by TUNEL assays, analyzed by flow cytometric. Figure 3B : The expression after transfection was tested on Western blots of non-transfected (nt), BARD1 transfected, or BARD1 Q564H mutant transfected cells. An increase of 97 kD BARD1 protein can be observed while expression of actin is constant. Transfection efficiency was tested by cotransfection of GFP and was at least 20 percent.
**Figure 4.** BARD1 induced apoptosis is independent of BRCA1.
   Transfection of pcDNA, BARD1, or BRCA1 expression plasmids was performed and apoptosis induction was monitored 24 hours after transfection. Efficiency of transfection was 20 percent as determined by transfection of GFP expressing plasmids. Figure 4A : Inhibition of BARD1-induced apoptosis by BRCA1 is presented in cotransfection experiments in ES cells. Transient transfection assays were performed with pcDNA, BARD1, BARD1 + BRCA1, or BRCA1. The extent of apoptosis induction was measured by TUNEL staining and monitored by flow cytometry. Results presented are mean x (TUNEL-fluorescence) ± range (n=2).
   Influence of BRCA1 on apoptosis induction by BARD1 was analyzed in Brca1-/-and wild type (wt) ES cells. Figure 4B : Apoptosis induction after doxorubicin treatment (5 µg/ml), under the same conditions was quantified by TUNEL assays of wild type and Brca1-/- ES cells. Results presented are mean x (TUNEL-fluorescence) ± range (n=2).

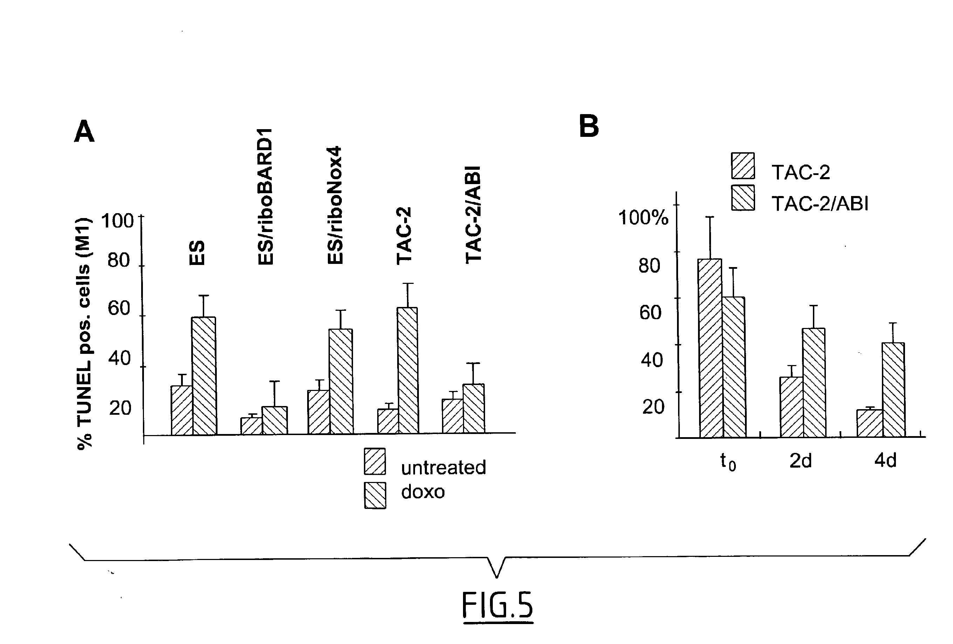

**Figure 5.** Repression of BARD1 reduces the apoptotic response to genotoxic stress.
   BARD1 repression was achieved by stable transfection of BARD1 ribozyme in ES cells (ES riboBARD1) or a BARD1 antisense in murine mammary cells (TAC-2/ABI). After treatment with doxorubicin (5 µg/ml, 6h), apoptosis was monitored by flow cytometric analysis of TUNEL staining. Comparison of results obtained for ES, ES/riboBARD1, TAC-2, TAC-2/ABI, or ES/riboNox4 (transfected with an unrelated ribozyme) is shown in Figure 5A with mean x (TUNEL-fluorescence) ± S.E. (n=3). Figure 5B : Cell numbers of TAC-2 cells, or BARD1-repressed TAC-2/ABI cells in the absence or presence of doxorubicin (10 µg/ml), were monitored after two and four days. Five fields of cells from each time point and each cell type were analyzed and the mean ± S.E. remaining adherent cells are shown.

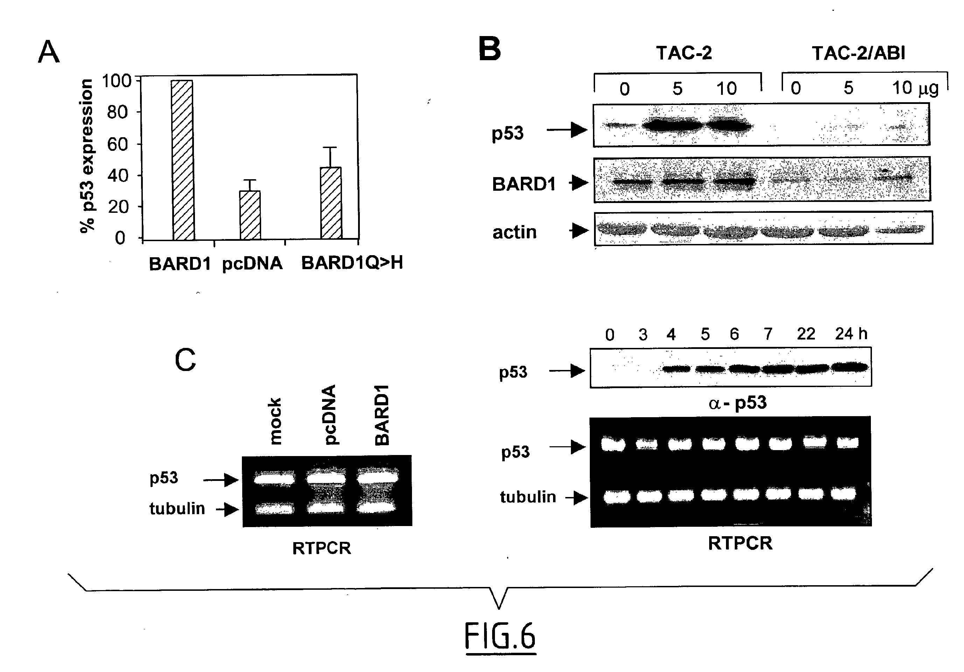

**Figure 6.** BARD1 induced apoptosis is associated with p53 accumulation.
   The p53 protein is upregulated in cells overexpressing BARD1 (Figure 6A). Expression of p53 protein in cells harvested 24 hours after transfection with indicated amounts of BARD1 and pcDNA3 or BARD1 was analyzed on Western blots. Effect of BARD1 and BARD1 Q564H mutant (BARD1 Q>H) on p53 expression were compared. While BARD1 increase is observed in BARD1 and BARD Q564H mutant transfected cells, significant increase of p53 is observed only in BARD1 transfected cells. The bar graph shows a comparison of p53 expression of cells transfected with pcDNA3 or BARD1Q564H mutant (BARD1Q>H), as a percentage of p53 expression in BARD1 transfected cells. Increase of p53 in response to doxorubicin is compared in ES and TAC-2 cells and in BARD1-repressed ES/riboBARD1 and TAC-2/ABI cells, and in control transfectants ES/riboNox4 (Figure 6B). Cells were incubated for 12 hours with doxorubicin (0, 5 or 10 µg/ml), and p53 and BARD1 accumulation was monitored by Western blotting. Unchanged levels of actin are shown. p53 upregulation is not due to transcriptional upregulation (figure 6C). p53 mRNA expression is tested by RT-PCR of RNA prepared from untransfected, pcDNA or BARD1 transfected cells (left panel). Protein (α-p53) and mRNA (RTPCR) expression are compared in TAC-2 cells after the indicated time of incubation with doxorubicin (right panel). Control of independent gene expression was performed with primers against mouse β-tubulin.

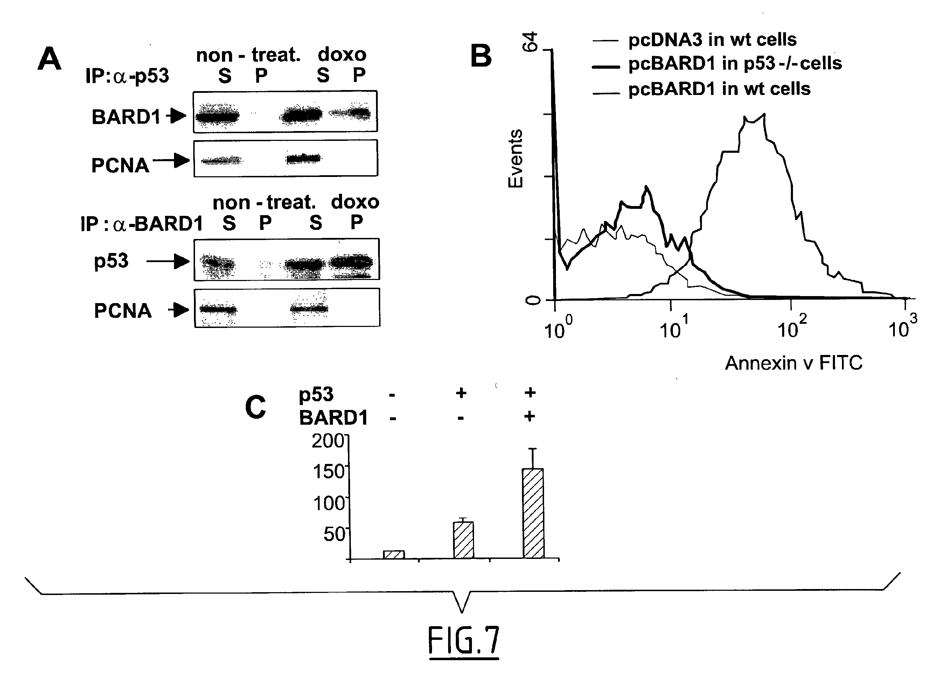

**Figure 7.** BARD1 induces apoptosis through interaction with functional p53. BARD1-p53 interaction was assayed by co-immunoprecipitation (figure A). Anti-p53 (agarose-coupled, Santa Cruz) or anti-BARD1 antibodies (N-19, Santa Cruz) were used for immunoprecipitations (IP) from non-treated (non-treat) and doxorubicin treated (doxo) cells, and Western blots were incubated with anti-BARD1 (PVC, Irminger-Finger et al., 1998) and anti-PCNA (Santa Cruz) or anti-p53, respectively. Supernatants (S) and pellet (P) fractions are presented. Figure 7B : BARD1 induced apoptosis depends on functional p53, as demonstrated by annexin V staining of wild type or p53 -/- ES cells transiently transfected with pcDNA3 or BARD1. BARD1 is required for p53 stabilization through protein-protein interaction as demonstrated by cotransfection of BARD1 and p53 in p53 deficient ES cells (Figure 7C), p53-/- ES cells were transfected with p53 or BARD1 plus p53. Increase of p53 was monitored on Western blots, and p53 protein bands were quantified. Bar graph presents intensities of band as arbitrary units.
**Figure 8.** Schematic presentation of BARD1 deletion mutants.
**Figure 9** : Model of dual functionality of BARD1 . Increase of BARD1 upon stress: pathway (1) BRCA1-BARD1 heterodimer formation, observed as nuclear dots (Scully et al., 1997), induction of DNA repair functions; pathway (2) BARD1 excess over BRCA1 (possibly as homodimers), BARD1 interaction with p53, affecting p53 stability and induction of apoptosis.

### EXAMPLES

### Example 1 : Apoptotic function of BARD1

### I - Experimental Procedures

### Western blotting and immunoprecipitation

For Western blots, protein extracts from tissues or cell cultures were prepared in standard lysis buffer (150 mM NaCl; 0.1% SDS; 50 mM Tris pH 8.0; 2 mM EDTA pH 8.0) and analyzed by Western blotting using anti-p53 (Santa Cruz FL-393), anti-BARD1 PVC (Irminger-Finger et al., 1998), N-19 and C-20 (Santa-Cruz sc-7373), or anti-β-tubulin (Boehringer) antibodies. Immuno precipitation was performed by homogenizing cell samples in 100 µl (100-150 µg total protein) RIPA buffer (150 mM NaCI; 1% NP-40; 0.5% NaDOC; 0.1% SDS; 50 mM Tris pH 8.0; 2 mM EDTA pH 8.0). Cell extracts were incubated with agarose-coupled p53 antibodies (Fl 393 Santa Cruz) in a 1 to 100 dilution for 2 hours, or BARD1 N-19 antibodies in 1 to 100 dilution over night, followed by incubation with sepharose-bound protein G for 2 hours. Extracts were centrifuged, pellets were washed twice with RIPA buffer and resuspended in 100 µl RIPA buffer. 25 µl of supernatant and pellet fractions were analyzed by Western blotting. Total protein content in supernatant and pellet fractions was compared by PonceauS staining after protein transfer and was 90 to 10 percent at least. Antibody reactive bands were visualized, scanned and quantified using Imagequant.

### Doxorubicin treatment and UV exposure

TAC-2, ES, or HeLa cells were grown to sub-confluence in 100 mm Petri dishes, exposed to UV light to reach 2.5 to 10 Jm⁻² and cultured for another 3 hours at 37°C before harvesting. Doxorubicin (10, 50, 100 µg/ml) was added to the culture medium for 6 to 12 hours before harvesting for apoptosis tests.

### Flow cytometry

Cells were permeabilized and fixed with CytoFix (Pharmingen), incubated with anti-BARD1 antibodies and FITC-coupled secondary antibodies for flow cytometric analysis on a Galaxy Pro flow cytometer (Dako, Switzerland).

### Generation of expression clones and cell lines

BARD1 expression clone was produced by cloning the coding sequence of the mouse BARD1 cDNA (Irminger-Finger et al., 1998) into the Kpnl and Notl sites of pcDNA3. The Q564H mutation was generated with Quick Change Site-directed mutagenesis kit (Stratagene). The murine BRCA1 cDNA, a generous gift of G. Lenoir, was cloned into the pcDNA3 vector.

Transfections were performed reproducibly with transfecten (Qiagen). 1 to 4 µg of DNA were transfected per 100 mm Petri dish. Transfection efficiency was between 20 and 30 percent as assayed by parallel transfection of GFP cloned into pcDNA3 and analyzed by flow cytometry and fluorescence microscopy.

### Generation of BARD1 antisense and ribozyme cell lines

The ribozyme-carrying mouse ES cells were generated by polyclonal selection for neomycin resistance, using a BARD1 specific ribozyme (Irminger-Finger et al., 1998). As control for non-specific ribozyme effects, mouse ES cells were transfected with an irrelevant ribozyme (riboNox4). The mouse mammary gland cell clone TAC-2/ABI, which expresses BARD1 antisense RNA (Irminger-Finger et al., 1998) also reduces BARD1 expression by 50 to 60 percent. The p53-deficient ES cells were described before (Lakin et al., 1999). Brca1-deficient ES cells were described in Aprelikova et al., 2001.

### RT-PCR

Total RNA was prepared from cell cultures with Qiagen purification kits. RT-PCR (Perkin Elmer) was performed in simultaneous one step or in two steps reactions, leading to identical results. BARD1 primers were designed to amplify region 1840 through 2360 of the BARD1 cDNA, and p53 primers were designed to amplify region 118 through 310 of the p53 cDNA sequence. The p53 coding sequence was cloned by RT-PCR from TAC-2 cells (Genbank accession #AY044188).

### Quantification of apoptosis

Apoptosis was monitored by several criteria, including changes in cellular morphology observed by light microscopy and nuclear morphology observed after DAPI staining; DNA laddering was performed with Quick Apoptotic DNA Laddering Kit (Biosource International), and annexin V staining and TUNEL assays were performed with kits from Molecular Probes, and analyses were carried out on DAKO Galaxy pro flow cytometer. Caspase 3 activity test were performed with kits from (Molecular Probes) and analyzed on a multi-label counter Victor from Perkin Elmer (Switzerland).

### II - Results

### BARD1 expression in response to genotoxic stress in vitro

The inventors first studied the response of various cell lines to proapoptotic stress. Addition of the DNA-damaging chemotherapeutic agent doxorubicin to mouse mammary epithelial cells TAC-2 (Soriano et al., 1995), not only led to an increase in cellular p53 levels, but also to marked elevations of cellular BARD1 levels (Fig. 1A). Similar increase of BARD1 in response to doxorubicin treatment was also observed in HeLa cells and human SH-SY5Y neuroblastoma cells, as well as in mouse embryonic stem (ES) cells (see below). To test whether cellular damage induced by means other than doxorubicin also resulted in an increase of BARD1, cells of different tissue origins were exposed to UV light. BARD1 was induced in HeLa cells and TAC-2, and mouse ES cells (Fig. 1B) after UV treatment and further incubation for 3 hours. The increase of BARD1 might be regulated by transcriptional activation, as is demonstrated by RT-PCR. Most strikingly in mouse ES cells no BARD1 mRNA could be detected in untreated cells, while BARD1 mRNA is clearly present after UV or doxorubicin treatment (Fig. 1C). These results suggest that BARD1 upregulation upon genotoxic stress is transcriptionally regulated.

### Elevated expression of BARD1 leads to apoptosis

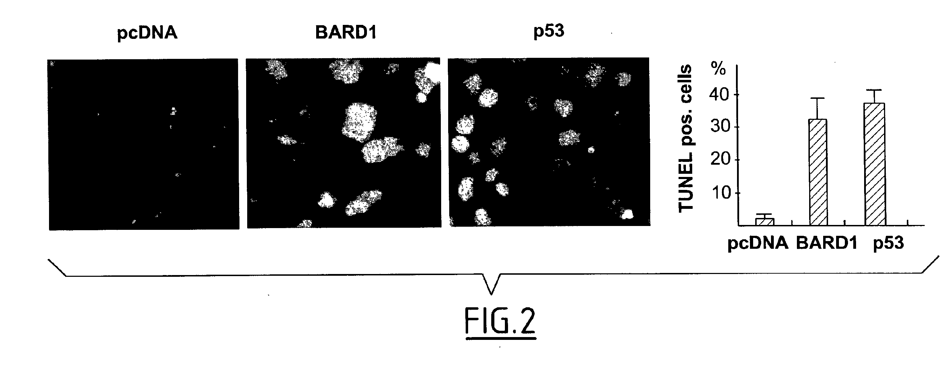

To address the question of whether BARD1 upregulation causes cell death, TAC-2 cells were transfected with a BARD1 expression plasmid. Attempts to establish stable BARD1-transfected cell lines were unsuccessful. Only mock-transfected (empty vector or GFP), but not BARD1-transfected cells yielded viable cell lines, suggesting deleterious effects of BARD1 overexpression. The inventors therefore analyzed transiently transfected cells. Transient transfection of TAC-2 cells with BARD1 led to apoptosis as assessed by TUNEL staining (Fig. 2), DNA laddering, caspase 3 activity tests, as well as annexin V staining and nuclear condensation monitored by DAPI staining. Transfection of cells with the *bona fide* proapoptotic protein p53 (Sionov and Haupt, 1999), led to a similar extent of apoptosis as transfection of BARD1. Control transfectants (empty vector or GFP) showed either no or only marginal signs of apoptosis. Thus, BARD1 transfection induced cell death, which displayed features of apoptosis. The inventors also observed apoptosis induced by BARD1 transfection in mouse ES, human SH-SY5Y cells, HeLa, and MCF-7 breast cancer cells albeit to different extents. Thus, BARD1 is a potent inducer of apoptosis in cells of different tissue or species origins. The relative increase of BARD1 protein levels observed after transient transfection were comparable to the increase after doxorubicin treatment, as assessed by Western blotting. However, since not all cells express BARD1 in a transient transfection assay, we also investigated BARD1 levels in individual cells by flow cytometry. Mean values of BARD1 levels in doxorubicin-treated cells were increased on average by a factor of 3.7 (± 2.4) with 5µg/ml doxorubicin, while mean values for BARD1 levels in BARD1-transfected cells increased 2.5 fold (± 1.8) over controls, showing a broader distribution. Thus, the mean cellular BARD1 levels are raised to comparable levels in doxorubicin-treated and BARD1-transfected cells.

To study the mechanisms of proapoptotic signaling by BARD1, the inventors analyzed the induction of apoptosis by a BARD1 deletion mutant and the Q564H missense mutation by monitoring apoptosis by DNA laddering and TUNEL assays (Fig. 3A). TAC-2 cells or mouse ES cells were transfected with pcDNA, BARD1 or the BARD1Q564H mutant. Transfection efficiency was standardized and the protein expression levels of exogenously expressed BARD1 and BARD1Q564H were comparable (Fig. 3B). DNA laddering and TUNEL assays were performed to monitor apoptosis induction. Interestingly, the BARD1Q564H point mutation was largely devoid of apoptosis-inducing activity. Since the Q564H mutation was shown to be associated with breast and endometrial cancer (Thai et al., 1998), its reduced apoptotic capacity provides a possible link between the tumor suppressor function of BARD1 and its proapoptotic activity.

### Apoptosis induction by BARD1 is independent of functional BRCA1

An involvement of BRCA1 in apoptotic signaling had been suggested previously (Harkin et al, 1999; Thangaraju et al., 2000). Thus, the BARD1 effects might be mediated through or require BRCA1. To investigate this question further, the inventors transfected BRCA1 into ES cells and into TAC-2 cells ; no apoptosis occurred under these conditions, while DNA laddering and positive TUNEL staining is observed after transfection of BARD1. Thus, overexpression of BRCA1 by itself does not induce apoptosis. To test whether BRCA1 synergized with BARD1 in apoptosis induction, we co-transfected BRCA1 and BARD1 (Fig. 4A). Similar increase of expression was monitored for BRCA1 and BARD1 after transfection. Interestingly, the cotransfection of BRCA1 diminished rather than enhanced apoptosis induction by BARD1, as determined by TUNEL staining and flow cytometry. To definitively clarify whether apoptosis induction by BARD1 occurs independently of BRCA1, the inventors studied apoptosis induction in Brca1-deficient mouse ES cells (Aprelikova et al., 2001). As assessed by DNA-laddering, BARD1 transfection induced apoptosis in Brca1-deficient cells as efficiently as doxorubicin (5 µg/ml). These results demonstrate unequivocally that BRCA1 is not required for BARD1-induced apoptosis.

When apoptosis was assessed quantitatively by TUNEL assay, the Brca1-deficient cells clearly showed increased sensitivity to apoptosis induction by both BARD1 and doxorubicin (Fig. 4B). Thus, cotransfection of BRCA1 diminishes BARD1-induced apoptosis, and the Brca1 -/- genetic background enhances BARD1-induced apoptosis. This suggests that BRCA1 actually antagonizes BARD1. Indeed, a BARD1 deletion mutant (RING finger deletion of amino acids 1 through 237) retains apoptosis inducing activity. Taken together, these results suggest that the BRCA1/BARD1 complex is not involved in apoptosis induction and that BARD1 must exert its proapoptotic activity either as a homodimer, or in a complex with other proteins.

### Repression of BARD1 reduces apoptotic response to genotoxic stress

So far the inventors have demonstrated that i) BARD1 levels increase in response to apoptotic stimuli, and ii) elevation of BARD1 levels is sufficient to induce apoptosis. To assess whether BARD1 is required for the induction of apoptosis, we studied the effect of BARD1 repression. For this purpose we used two different systems for BARD1 repression, ribozymes and antisense RNA. Mouse ES cells stably expressing anti-BARD1 ribozymes were generated, and TAC-2 cells stably expressing BARD1 antisense RNA were described previously (TAC-2/ABI; Irminger-Finger et al., 1998). In both systems, doxorubicin (5 µg/ml, 6 h) induced much less apoptosis (i.e. TUNEL-positive cells), as compared to control cells or cells transfected with an irrelevant ribozyme (Fig. 5A). Resistance to doxorubicin-induced apoptosis through BARD1 antisense expression could also be monitored by simple cell count (Fig. 5B) or by measuring caspase 3 activity. Caspase 3 activity (Molecular Probes) was measured by fluorometry after treatment with doxorubicin (10 µg/ml) for the indicated periods of time. Results are mean ± S.E. (n=3). Experiments were performed in the presence and absence of the caspase 3 inhibitors DEVD.

In control cells doxorubicin led to almost 100 percent cell death within a few days, whereas BARD1-repressed cells showed delayed and reduced response. Taken together, these results suggest that the apoptotic response to doxorubicin is, at least in part, mediated through BARD1.

### Apoptosis induction by BARD1 affects p53 protein accumulation

To further study the mechanism of BARD1-induced apoptosis, the inventors investigated the relationship between BARD1 and the p53 pathway of apoptosis. Transfection of BARD1 into TAC-2 cells, that express wild type p53 (see Experimental Procedures; RT-PCR) led to a marked increase in p53 protein levels (Fig. 6A), significantly higher than after transfection of vector sequences (Fig. 6A) or expression of unrelated proteins (not shown). Importantly, transfection of the BARD1 mutation Q564H, which showed reduced apoptotic capacity when expressed in TAC-2 cells or ES cells (Fig. 3), resulted in significantly less p53 accumulation than obtained with wild type BARD1 (Fig. 6A).

If BARD1 was a relevant link between DNA damage and p53 elevations, a decreased expression of p53 in BARD1-suppressed cells could be expected. Indeed, BARD1 repression led to reduced p53 elevations in doxorubicin-treated cells, as shown for the two cell lines described above (Fig. 6B). Transfection of an irrelevant ribozyme (Nox4) did not alter p53 induction (Fig. 6B). The upregulation of p53 observed at the protein level could be due to transcriptional upregulation of p53. RT-PCR was performed to determine the changes in the p53 mRNA levels. No significant differences, neither of p53 mRNA nor of the control house keeping gene β-tubulin, were observed in mock-transfected or pcDNA-transfected cells when compared to BARD1-transfected cells (Fig.6C). To further analyze p53 accumulation, the time course of expression of p53 protein and p53 mRNA after doxorubicin treatment were compared. While p53 protein levels were undetectable in untreated cells and increased after a few hours of treatment, p53 mRNA levels, as detectable by RT-PCR were elevated in untreated cells, and did not increase significantly after exposure to doxorubicin (Fig. 6C). It is therefor unlikely that BARD1 and doxorubicin-induced p53 protein accumulation are due to transcriptional upregulation. It rather appears that BARD1 exerts its action on p53 at a posttranslational level, consistent with the concept of posttranslational regulation of p53 levels during apoptosis (Lakin et al., 1999).

### BARD1-p53 interaction is required for BARD1 induced apoptosis

To determine how BARD1 induces p53 accumulation, we investigated whether the two proteins interact physically by performing co-immunoprecipitation experiments (Fig. 7A). Cell lysates from TAC-2 cells (Fig. 7A-C), mouse ES cells, or HeLa cells, untreated or after treatment with doxorubicin, were tested. When using either anti-BARD1 antibodies or anti-p53 antibodies, BARD1 and p53 co-immunoprecipitated. Using p53 antibodies, 36 percent (+/- 4; n=2) of BARD1 were found in the pellet after treatment with doxorubicin (Fig. 7A) and p53 co-immunprecipitation with BARD1 antibodies resulted in 52 percent (+/- 3; n=2) of the protein in the pellet fraction after doxorubicin treatment (Fig. 7A). The nuclear protein PCNA, shown previously to be associated with BRCA1 in DNA repair functions (Scully et al. 1997) did not co-immunoprecipitate under our experimental conditions, demonstrating specificity of the p53-BARD1 interaction. However, this co-immunoprecipitation was observed only in doxorubicin-treated cells. The lack of co-immunoprecipitation of BARD1 and p53 in non-treated cells may simply be due to the very low p53 protein levels in non-treated cells. The interaction between BARD1 and p53 was also observed in TAC-2 cells in which p53 elevations were induced by BARD1 transfection. Efficiency of BARD1-p53 interaction was compared in transient transfections of BARD1 or BARD1Q564H (BARD1Q>H) mutant. Co-immunoprecipitation was markedly decreased for the BARD1 mutant Q564H. Only 19 percent (+/- 2; n=3) of p53 co-immunprecipitated with BARD1 Q564H, while cells transfected with wild type BARD1 showed 58 percent of p53 protein in the pellet fraction. Thus, the tumor-associated BARD1 Q564H mutant interacts less avidly with p53 than wild type BARD1.

To further ascertain the role of p53 in BARD1-induced apoptosis, the inventors investigated the apoptotic activity of BARD1 in p53-deficient mouse ES cells (Corbet et al., 1999). Transfection of BARD1 into p53-/- cells did not lead to apoptosis, as determined by annexin V staining (Fig. 7B) and DNA ladder assay, while apoptosis was induced in the corresponding wild type cells. Thus, p53 is required for BARD1-induced apoptosis.

The data presented so far demonstrate that increase of BARD1 leads to an increase of p53 protein which is not accompanied by detectable elevations of p53 mRNA. Together with the direct protein-protein interaction of the two molecules shown above, this suggests postranslational stabilization of p53 by BARD1. To further substantiate this point, p53-deficient ES cells were transfected with vector sequence, with p53 plus control vector, or with p53 plus BARD1. In this system p53 (expressed from the constitutive CMV promoter) cannot be controlled on a transcriptional level, but p53 protein stabilization must result from interaction with BARD1. Indeed, as shown in Fig. 7C, markedly higher levels of p53 protein are observed in cells co-transfected with p53 and BARD1, than in cells transfected with p53 only.

### III - Discussion

These results demonstrate a function of BARD1 in signaling between genotoxic stress and induction of apoptosis: BARD1 levels increase in response to genotoxic stress and are necessary and sufficient for p53 upregulation that results in apoptotic response (Sionov and Haupt, 1999).

### Relationship between BRCA1 and BARD1-induced apoptosis

Hitherto described BARD1 functions depend on its association with BRCA1 and a function of BRCA1 in apoptosis induction was proposed previously (Harkin et al., 1999). However, the function of BARD1 as apoptosis inducer, described here, has to be attributed to BARD1 independently of is association with BRCA1, because: i) only BARD1, but not BRCA1 increased after stroke; ii) the co-transfection of BRCA1 did not enhance BARD1-induced apoptosis; iii) BARD1 induced apoptosis in BRCA1-deficient cells. Thus, BRCA1 is not required for apoptosis induction by BARD1. Our results even indicate that the interaction of BRCA1 with BARD1 reduces apoptosis induction, as BARD1-induced apoptosis is diminished by co-transfection of BRCA1, but enhanced in BRCA1-deficient cells.

### Mechanisms of BARD1-induced p53 elevations

These results further demonstrate that DNA damage leads to upregulation of BARD1, presumably by altering RNA levels (Fig. 1C). Increase of BARD1 protein subsequently affects p53 protein stabilization through a mechanism that most likely involves direct BARD1-p53 protein-protein interaction. The most straightforward explanation of biochemical mechanisms sustaining the BARD1 induced p53 upregulation would be BARD1-induced changes of the p53 molecule leading to protein stabilization. Alternatively however, it might be due to an interference of BARD1 with ubiquitin-dependent p53 degradation. Indeed, p53 turnover is controlled by the cellular protein degradation machinery involving ubiquitin ligases, such as E6-AP (Scheffner et al., 1993; Rodriguez et al., 2000) and MDM-2. Several observations point towards a role of BARD1 in ubiquitination: BARD1 possesses an N-terminal RING finger domain found in many proteins implicated in ubiquitination (Freemont, 2000), and as recently shown, the BARD1/BRCA1 complex possesses ubiquitin ligase activity (Scully and Livingston, 2000; Hashizume et al., 2001). Our data do not exclude that BARD1 alters p53 ubiquitination; however, as BARD1 induction of apoptosis is BRCA1-independent, ubiquitination would have to occur through mechanisms others than those described (Hashizume et al., 2001). Also, it is very unlikely that the recently described function of BARD1 in inhibition of mRNA 3'end processing (Kleiman and Manley, 2001) is linked to the BARD1-induced apoptosis pathway described here, since it involves the formation of a BARD1/BRCA1/CstF-50 complex.

### Example 2 : Characterization of BARD1 protein regions required for apoptotic activity

The inventors have generated EGFP-BARD1 fusion constructs under the transcriptional control of the GFP promoter and expressed them in a variety of cell types. Under these conditions full length BARD1-GFP locates to the nucleus and to the cytoplasm. However the deletion mutant ΔRING-GFP localizes exclusively to the nucleus. More surprisingly a mutant bearing a C-terminal deletion, ΔBRCT-GFP, localizes to distinct dots within the nucleus.

Apoptotic activity of deletion BARD1-EGFP was compared to the activity of pcBARD1 and found to be as active as pcBARD1. Control transfection of pcEGFP did not induce significant levels of apoptosis (Fig. 8). pcBARD-ΔRING-EGFP showed similar levels of apoptosis as BARD1-EGFP but the onset of apoptosis was delayed. Deletion mutant ΔBRCT-GFP did not induce significant levels of apoptosis.

The BARD1 RING finger motif is not required for nuclear localization nor for apoptotic activity. However, the BRCT domain or/and the upstream adjacent region of BARD1 are required for apoptosis induction.

### REFERENCES

Abbott et al., (1999) J. Biol. Chem. 274, 18808-18812

Akli et al. (1993) Nature Genetics 3 224

Aprelikova et al., (2001) J. Biol. Chem. 276(28):25647-50.

Ausubel *et al.* (1994) *Current Protocols in Molecular Biology*

Benoist et al. (1981) Nature,290:304-310

Bienstock R. J., Darden T., Wiseman R., Pedersen L., Barrett J. C. (1996). Molecular modeling of the amino-terminal zinc ring domain of BRCA1. Cancer Res. 56, 2539-2545.

Brinster et al. (1982) Nature, 296:39-42

Chen et al. (1995) Science, 270, 789-791

Chiocca et al. (1990) New Biol. 2 739

Corbet S. W., Clarke A. R., Gledhill S., Wyllie A.H. (1999). P53-dependent and -independent links between DNA-damage, apoptosis and mutation frequency in ES cells. Oncogene 18, 1537-1544.

Danos et al. (1988) PNAS 85 6460

DeBoer et al. (1980) "Useful proteins from recombinant bacteria" in Scientific American, 242:74-94

DeBoer et al. (1983) Proc. Natl. Acad. Sci. USA, 80:21-25

Dengler et al. (1995) Anticancer Drugs. 6:522-32

Dobson et al. (1990) Neuron 5 353

Felgner et al. (1989) Science 337:387-388

Felgner, et. al. (1987) Proc. Natl. Acad. Sci. U.S.A. 84:7413-7417

Ferenczy A. (1993). Ultrastructure of the normal menstrual cycle. Microsc Res Tech. 25, 91-105.

Freemont P.S. (2000). RING for destruction? Curr. Biol. 10, 84-87.

Freshney (1986) *Animal Cell Culture; Immobilized Cells and Enzymes*

Gautier F., Irminger-Finger I., Gregoire **M.,** Meflah K., Harb J. (2000). Identification of an apoptotic cleavage product of BARD1 as an autoantigen: a potential factor in the antitumoral response mediated by apoptotic bodies. Cancer Res. 60, 6895-6900.

Gorczyca (1993) Cancer Res 53:1945-51.

Gowen et al, (1998) Science, 281, 1009-1012

Graham (1984) EMBO J. 3(122917)

Graham et al. (1977) J. Gen. Virol. 36: 59

Hames et al. (1984) *Transcription and Translation*

Harkin D. P., Bean J. M., Miklos D., Song Y. H., Truong V. B., Englert C., Christians F. C., Ellisen L. W., Maheswaran S., Oliner J. D., Haber D. A. (1999). Induction of GADD45 and JNK/SAPK-dependent apoptosis following inducible expression of BRCA1. Cell 97, 575-586.

Hashizume et al., (2001) J. Biol. Chem. 276(18):14537-40

Irminger-Finger I., Soriano J. V., Vaudan G., Montesano R., Sappino A. P. (1998). In vitro repression of Brca1-associated RING domain gene, Bard1, induces phenotypic changes in mammary epithelial cells. J Cell Biol. 143, 1329-1339.

Joukov et al. (2001) Functional communication between endogenous BRCA1 and its partner, BARD1, during Xenopus laevis development. Proc Natl Acad Sci U S A.;98(21):12078-83.

Kleiman F. E., Manley J. L. (1999). Functional interaction of BRCA1-associated BARD1 with polyadenylation factor CstF-50. Science 285, 1576-1579.

Kleiman F. E., Manley J. L. (2001). The BARD1-CstF-50 interaction links mRNA 3'end formation to DNA damage and tumor suppression. Cell 104, 743-753.

Kollias et al. (1986) Cell, 46: 89-94

Lakin N. D., Jackson S. P. (1999). Regulation of p53 in response to DNA damage. Oncogene. 18, 7644-7655.

Le Gal la Salle et al. (1993) Science 259 988

Levrero et al., (1991) Gene 101 195

Mackey, et al., (1988) Proc. Natl. Acad. Sci. U.S.A. 85:8027-8031

Maouche et al. (1991) Blood, 15:2557

Meza J. E., Brzovic P. S., King M. C., Klevit R. E. (1999). Mapping the functional domains of BRCA1. Interaction of the ring finger domains of BRCA1 and BARD1. J. Biol. Chem. 274, 5659-5665.

Miki Y., Swensen J., Shattuck-Eidens D., Futreal P. A., Harshman K., Tavtigian S., Liu Q., Cochran C., Bennett L. M., Ding W., et al. (1994). A strong candidate for the breast and ovarian cancer susceptibility gene BRCA1. Science 266, 66-71.

Miyanohara et al., (1992) New Biol. 4 238

Mogram et al. (1985) Nature, 315:338-340

Paull et al, (2001) Proc. Natl. Acad. Sci. USA 98, 6086-6091

Perbal, (1984) *A Practical Guide To Molecular Cloning*

Raff, M. (1998). Nature. 396:119-122

Reeck et al. (1987) Cell 50:667

Rodriguez M. S., Desterro J. M., Lain S., Lane D. P., Hay R. T. (2000). Multiple C-terminal lysine residues target p53 for ubiquitin-proteasome-mediated degradation. Mol. Cell Biol. 20, 8458-8467.

Roemer et al. (1992) Eur. J. Biochem. 208 211

Sambrook et al., (1989) *Molecular Cloning: A Laboratory Manual,* Second Edition Cold Spring Harbor Laboratory Press, Cold Spring Harbor, New York

Scheffner M., Huibregtse J. M., Vierstra R. D., Howley P. M. (1993). The HPV-16 E6 and E6-AP complex functions as a ubiquitin-protein ligase in the ubiquitination of p53. Cell 75, 495-505.

Scully R., Chen J., Ochs R. L., Keegan K., Hoekstra M., Feunteun J., Livingston D. M. (1997). Dynamic changes of BRCA1 subnuclear location and phosphorylation state are initiated by DNA damage. Cell 90, 425-435.

Scully R., Livingston D. M. (2000). In search of the tumour-suppressor functions of BRCA1 and BRCA2. Nature 408, 429-432.

Sionov R. V., Haupt Y. (1999). The cellular response to p53: the decision between life and death. Oncogene 18, 6145-6157.

Soriano J. V., Pepper M. S., Nakamura T., Orci L., Montesano R. (1995). Hepatocyte growth factor stimulates extensive development of branching duct-like structures by cloned mammary gland epithelial cells. J. Cell Sci. 108, 413-430.

Stratford-Perricaudet et al. (1990) Human *Gene Therapy* 1 241

Thai T. H., Du F., Tsan J. T., Jin Y., Phung A., Spillman M. A., Massa H. F., Muller C. Y., Ashfaq R., Mathis J. M., Miller D. S., Trask B. J., Baer R., Bowcock A. M. (1998). Mutations in the BRCA1-associated RING domain (BARD1) gene in primary breast, ovarian and uterine cancers. Hum. Mol. Genet. 7, 195-202.

Thangaraju M., Kaufmann S. H., Couch F. J. (2000). BRCA1 facilitates stress-induced apoptosis in breast and ovarian cancer cell lines. J. Biol. Chem. 275, 33487-33496.

Villa-Komaroff et al. (1978) Proc. Natl. Acad. Sci. USA,75:3727-3731)

Vogelstein (1990) Nature, 348:681

Wagner et al. (1981) Proc. Natl. Acad. Sci. USA, 78: 1441-1445

Wang et al., (2000) Genes. Dev., 14, 927-939

Wu et al. (1988) J. Biol. Chem. 263:14621-14624

Wu et al., (1992) J. Biol. Chem. 267:963-967

Wu L. C., Wang Z. W., Tsan J. T., Spillman M. A., Phung A., Xu X. L., Yang M. C., Hwang L. Y., Bowcock A. M., Baer R. (1996). Identification of a RING protein that can interact in vivo with the BRCA1 gene product. Nat. Genet. 14, 430-440.

Yamamoto et al. (1980) Cell, 22:787-797,

Yoshikawa K., Ogawa T., Baer R., Hemmi H., Honda K., Yamauchi A., Inamoto T. Ko K., Yazumi S., Motoda H., Kodama H., Noguchi S., Gazdar A. F., Yamaoka Y., Takahashi R. (2000). Abnormal expression of BRCA1 and BRCA1-interactive DNA-repair proteins in breast carcinomas. Int. J. Cancer 88, 28-36.

Zhong et al, (1999) Science, 285, 747-750

## Claims

1. Use of a BARD1 encoding nucleic acid in the manufacture of a medicament useful for inducing apoptosis of an undesired cell.

2. The use according to claim 1, which the nucleic acid encodes a BARD1 protein comprising an amino acid sequence shown as SEQ ID NO:2.

3. The use according to claim 1 or 2 , wherein the nucleic acid is part of a viral vector.

4. The use according to any of claims 1 to 3, wherein the BARD1 encoding nucleic acid is associated with a p53 encoding nucleic acid for the manufacture of a therapeutic association useful for inducing apoptosis of an undesired cell.

5. The use according to claim 4, wherein the BARD1 encoding nucleic acid is part of a vector that further encodes a p53 gene product.

6. The use according to any of claims 1 to 3, wherein the BARD1 encoding nucleic acid is associated with a p53 gene product or an agent that stimulates p53 expression or activity for the manufacture of a therapeutic association useful for inducing apoptosis of an undesired cell.

7. The use according to any of claims 1 to 6, wherein the BARD1 encoding nucleic acid is associated with an agent that inhibits BRCA1 expression.

8. Use of a BARD1 protein or a BARD1 peptide that shows the BARD1 ability to interact with p53, in the manufacture of a medicament useful for inducing apoptosis of an undesired cell.

9. Use of an agent that stimulates BARD1 endogenous expression or activity in the manufacture of a medicament useful for inducing apoptosis of an undesired cell.

10. The use according to any of claims 1 to 9, wherein the cell is a tumor cell.

11. The use according to any of claims 1 to 9, wherein the cell is a cell infected with a pathogenic agent.

12. An expression vector that comprises
i) a BARD1 encoding nucleic acid ; and
ii) a p53 encoding nucleic acid,
said nucleic acids being operatively associated with expression control sequences.

13. A pharmaceutical composition comprising the expression vector of claim 12, in association with a pharmaceutically acceptable carrier.

14. A pharmaceutical composition comprising :
- an expression vector that comprises a BARD1 encoding nucleic acid operatively associated with expression control sequences ; and
- an expression vector that comprises a p53 encoding nucleic acid operatively associated with expression control sequences ;
in association with a pharmaceutically acceptable carrier.

15. A kit comprising :
- in a first container, a pharmaceutical composition comprising an expression vector that comprises a BARD1 encoding nucleic acid operatively associated with expression control sequences ; and
- in a second container, a pharmaceutical composition comprising an expression vector that comprises a p53 encoding nucleic acid operatively associated with expression control sequences.

16. A pharmaceutical composition comprising :
i) a BARD1 encoding nucleic acid, and
ii) a BRCA1 antisense nucleic acid ;
in association with a pharmaceutically acceptable carrier.

17. A kit comprising :
- in a first container, a pharmaceutical composition comprising an expression vector that comprises a BARD1 encoding nucleic acid operatively associated with expression control sequences ; and
- in a second container, a pharmaceutical composition comprising a BRCA1 antisense nucleic acid.


SEQUENCE LISTING

<110> Universite de Geneve
      INSERM

<120> A method for inducing apoptosis

<130> BET 01/1235

<140>
<141>

<160> 2

<170> PatentIn Ver. 2.1

<210> 1
<211> 2530
<212> DNA
<213> Homo sapiens

<220>
<221> CDS
<222> (74)..(2407)

<400> 1
cagcttccct gtggtttccc gaggcttcct tgcttcccgc tctgcgagga gcctttcatc 60

cgaaggcggg acg atg ccg gat aat cgg cag ccg agg aac cgg cag ccg    109
               Met Pro Asp Asn Arg Gln Pro Arg Asn Arg Gln Pro
                 1               5                  10

agg atc cgc tcc ggg aac gag cct cgt tcc gcg ccc gcc atg gaa ccg    157
Arg Ile Arg Ser Gly Asn Glu Pro Arg Ser Ala Pro Ala Met Glu Pro
         15                  20                  25

gat ggt cgc ggt gcc tgg gcc cac agt cgc gcc gcg ctc gac cgc ctg    205
Asp Gly Arg Gly Ala Trp Ala His Ser Arg Ala Ala Leu Asp Arg Leu
     30                  35                  40

gag aag ctg ctg cgc tgc tcg cgt tgt act aac att ctg aga gag cct    253
Glu Lys Leu Leu Arg Cys Ser Arg Cys Thr Asn Ile Leu Arg Glu Pro
 45                  50                  55                  60

gtg tgt tta gga gga tgt gag cac atc ttc tgt agt aat tgt gta agt    301
Val Cys Leu Gly Gly Cys Glu His Ile Phe Cys Ser Asn Cys Val Ser
                 65                  70                  75

gac tgc att gga act gga tgt cca gtg tgt tac acc ccg gcc tgg ata    349
Asp Cys Ile Gly Thr Gly Cys Pro Val Cys Tyr Thr Pro Ala Trp Ile
             80                  85                  90

caa gac ttg aag ata aat aga caa ctg gac agc atg att caa ctt tgt    397
Gln Asp Leu Lys Ile Asn Arg Gln Leu Asp Ser Met Ile Gln Leu Cys
         95                 100                 105

agt aag ctt cga aat ttg cta cat gac aat gag ctg tca gat ttg aaa    445
Ser Lys Leu Arg Asn Leu Leu His Asp Asn Glu Leu Ser Asp Leu Lys
    110                 115                 120

gaa gat aaa cct agg aaa agt ttg ttt aat gat gca gga aac aag aag    493
Glu Asp Lys Pro Arg Lys Ser Leu Phe Asn Asp Ala Gly Asn Lys Lys
125                 130                 135                 140

aat tca att aaa atg tgg ttt agc cct cga agt aag aaa gtc aga tat    541
Asn Ser Ile Lys Met Trp Phe Ser Pro Arg Ser Lys Lys Val Arg Tyr

2

145                    150 155

gtt gtg agt aaa gct tca gtg caa acc cag cct gca ata aaa aaa gat    589
Val Val Ser Lys Ala Ser Val Gln Thr Gln Pro Ala Ile Lys Lys Asp
            160                 165                 170

gca agt gct cag caa gac tca tat gaa ttt gtt tcc cca agt cct cct    637
Ala Ser Ala Gln Gln Asp Ser Tyr Glu Phe Val Ser Pro Ser Pro Pro
        175                 180                 185

gca gat gtt tct gag agg gct aaa aag gct tct gca aga tct gga aaa    685
Ala Asp Val Ser Glu Arg Ala Lys Lys Ala Ser Ala Arg Ser Gly Lys
    190                 195                 200

aag caa aaa aag aaa act tta gct gaa atc aac caa aaa tgg aat tta    733
Lys Gln Lys Lys Lys Thr Leu Ala Glu Ile Asn Gln Lys Trp Asn Leu
205                 210                 215                 220

gag gca gaa aaa gaa gat ggt gaa ttt gac tcc aaa gag gaa tct aag    781
Glu Ala Glu Lys Glu Asp Gly Glu Phe Asp Ser Lys Glu Glu Ser Lys
                225                 230                 235

caa aag ctg gta tcc ttc tgt agc caa cca tct gtt atc tcc agt cct    829
Gln Lys Leu Val Ser Phe Cys Ser Gln Pro Ser Val Ile Ser Ser Pro
            240                 245                 250

cag ata aat ggt gaa ata gac tta cta gca agt ggc tcc ttg aca gaa    877
Gln Ile Asn Gly Glu Ile Asp Leu Leu Ala Ser Gly Ser Leu Thr Glu
        255                 260                 265

tct gaa tgt ttt gga agt tta act gaa gtc tct tta cca ttg gct gag    925
Ser Glu Cys Phe Gly Ser Leu Thr Glu Val Ser Leu Pro Leu Ala Glu
    270                 275                 280

caa ata gag tct cca gac act aag agc agg aat gaa gta gtg act cct    973
Gln Ile Glu Ser Pro Asp Thr Lys Ser Arg Asn Glu Val Val Thr Pro
285                 290                 295                 300

gag aag gtc tgc aaa aat tat ctt aca tct aag aaa tct ttg cca tta    1021
Glu Lys Val Cys Lys Asn Tyr Leu Thr Ser Lys Lys Ser Leu Pro Leu
                305                 310                 315

gaa aat aat gga aaa cgt ggc cat cac aat aga ctt tcc agt ccc att    1069
Glu Asn Asn Gly Lys Arg Gly His His Asn Arg Leu Ser Ser Pro Ile
            320                 325                 330

tct aag aga tgt aga acc agc att ctg agc acc agt gga gat ttt gtt    1117
Ser Lys Arg Cys Arg Thr Ser Ile Leu Ser Thr Ser Gly Asp Phe Val
        335                 340                 345

aag caa acc gtg ccc tca gaa aat ata cca ttg cct gaa tgt tct tca    1165
Lys Gln Thr Val Pro Ser Glu Asn Ile Pro Leu Pro Glu Cys Ser Ser
    350                 355                 360

cca cct tca tgc aaa cgt aaa gtt ggt ggt aca tca ggg agg aaa aac    1213
Pro Pro Ser Cys Lys Arg Lys Val Gly Gly Thr Ser Gly Arg Lys Asn
365                 370                 375                 380

agt aac atg tcc gat gaa ttc att agt ctt tca cca ggt aca cca cct    1261
Ser Asn Met Ser Asp Glu Phe Ile Ser Leu Ser Pro Gly Thr Pro Pro
                385                 390                 395

tct aca tta agt agt tca agt tac agg caa gtg atg tct agt ccc tca    1309
Ser Thr Leu Ser Ser Ser Ser Tyr Arg Gln Val Met Ser Ser Pro Ser

3

400                     405     410

gca atg aag ctg ttg ccc aat atg gct gtg aaa aga aat cat aga gga    1357
Ala Met Lys Leu Leu Pro Asn Met Ala Val Lys Arg Asn His Arg Gly
        415                 420                 425

gag act ttg ctc cat att gct tct att aag ggc gac ata cct tct gtt    1405
Glu Thr Leu Leu His Ile Ala Ser Ile Lys Gly Asp Ile Pro Ser Val
    430                 435                 440

gaa tac ctt tta caa aat gga agt gat cca aat gtt aaa gac cat gct    1453
Glu Tyr Leu Leu Gln Asn Gly Ser Asp Pro Asn Val Lys Asp His Ala
445                 450                 455                 460

gga tgg aca cca ttg cat gaa gct tgc aat cat ggg cac ctg aag gta    1501
Gly Trp Thr Pro Leu His Glu Ala Cys Asn His Gly His Leu Lys Val
                465                 470                 475

gtg gaa tta ttg ctc cag cat aag gca ttg gtg aac acc acc ggg tat    1549
Val Glu Leu Leu Leu Gln His Lys Ala Leu Val Asn Thr Thr Gly Tyr
            480                 485                 490

caa aat gac tca cca ctt cac gat gca gcc aag aat ggg cac gtg gat    1597
Gln Asn Asp Ser Pro Leu His Asp Ala Ala Lys Asn Gly His Val Asp
        495                 500                 505

ata gtc aag ctg tta ctt tcc tat gga gcc tcc aga aat gct gtt aat    1645
Ile Val Lys Leu Leu Leu Ser Tyr Gly Ala Ser Arg Asn Ala Val Asn
    510                 515                 520

ata ttt ggt ctg cgg cct gtc gat tat aca gat gat gaa agt atg aaa    1693
Ile Phe Gly Leu Arg Pro Val Asp Tyr Thr Asp Asp Glu Ser Met Lys
525                 530                 535                 540

tcg cta ttg ctg cta cca gag aag aat gaa tca tcc tca gct agc cac    1741
Ser Leu Leu Leu Leu Pro Glu Lys Asn Glu Ser Ser Ser Ala Ser His
                545                 550                 555

tgc tca gta atg aac act ggg cag cgt agg gat gga cct ctt gta ctt    1789
Cys Ser Val Met Asn Thr Gly Gln Arg Arg Asp Gly Pro Leu Val Leu
            560                 565                 570

ata ggc agt ggg ctg tct tca gaa caa cag aaa atg ctc agt gag ctt    1837
Ile Gly Ser Gly Leu Ser Ser Glu Gln Gln Lys Met Leu Ser Glu Leu
        575                 580                 585

gca gta att ctt aag gct aaa aaa tat act gag ttt gac agt aca gta    1885
Ala Val Ile Leu Lys Ala Lys Lys Tyr Thr Glu Phe Asp Ser Thr Val
    590                 595                 600

act cat gtt gtt gtt cct ggt gat gca gtt caa agt acc ttg aag tgt    1933
Thr His Val Val Val Pro Gly Asp Ala Val Gln Ser Thr Leu Lys Cys
605                 610                 615                 620

atg ctt ggg att ctc aat gga tgc tgg att cta aaa ttt gaa tgg gta    1981

                625                 630                 635

aaa gca tgt cta cga aga aaa gta tgt gaa cag gaa gaa aag tat gaa    2029

            640                 645                 650

att cct gaa ggt cca cgc aga agc agg ctc aac aga gaa cag ctg ttg    2077


4

655                 660                 665

cca aag ctg ttt gat gga tgc tac ttc tat ttg tgg gga acc ttc aaa     2125

    670                 675                 680

cac cat cca aag gac aac ctt att aag ctc gtc act gca ggt ggg ggc     2173

685                 690                 695                 700

cag atc ctc agt aga aag ccc aag cca gac agt gac gtg act cag acc     2221

                705                 710                 715

atc aat aca gtc gca tac cat gcg aga ccc gat tct gat cag cgc ttc     2269

            720                 725                 730

tgc aca cag tat atc atc tat gaa gat ttg tgt aat tat cac cca gag     2317

        735                 740                 745

agg gtt cgg cag ggc aaa gtc tgg aag gct cct tcg agc tgg ttt ata     2365

    750                 755                 760

gac tgt gtg atg tcc ttt gag ttg ctt cct ctt gac agc tga             2407

765                 770                 775

atattatacc agatgaacat ttcaaattga atttgcacgg tttgtgagag cccagtcatt 2467

gtactgtttt taatgttcac atttttacaa ataggtagag tcattcatat ttgtctttga 2527

atc                                                               2530

<210> 2
<211> 777
<212> PRT
<213> Homo sapiens

<400> 2
Met Pro Asp Asn Arg Gln Pro Arg Asn Arg Gln Pro Arg Ile Arg Ser
1               5                   10                  15

Gly Asn Glu Pro Arg Ser Ala Pro Ala Met Glu Pro Asp Gly Arg Gly
            20                  25                  30

Ala Trp Ala His Ser Arg Ala Ala Leu Asp Arg Leu Glu Lys Leu Leu
        35                  40                  45

Arg Cys Ser Arg Cys Thr Asn Ile Leu Arg Glu Pro Val Cys Leu Gly
    50                  55                  60

Gly Cys Glu His Ile Phe Cys Ser Asn Cys Val Ser Asp Cys Ile Gly
65                  70                  75                  80

Thr Gly Cys Pro Val Cys Tyr Thr Pro Ala Trp Ile Gln Asp Leu Lys
                85                  90                  95

Ile Asn Arg Gln Leu Asp Ser Met Ile Gln Leu Cys Ser Lys Leu Arg
            100                 105                 110

5

Asn Leu Leu His Asp Asn Glu Leu Ser Asp Leu Lys Glu Asp Lys Pro
        115                 120                 125

Arg Lys Ser Leu Phe Asn Asp Ala Gly Asn Lys Lys Asn Ser Ile Lys
    130                 135                 140

Met Trp Phe Ser Pro Arg Ser Lys Lys Val Arg Tyr Val Val Ser Lys
145                 150                 155                 160

Ala Ser Val Gln Thr Gln Pro Ala Ile Lys Lys Asp Ala Ser Ala Gln
                165                 170                 175

Gln Asp Ser Tyr Glu Phe Val Ser Pro Ser Pro Pro Ala Asp Val Ser
            180                 185                 190

Glu Arg Ala Lys Lys Ala Ser Ala Arg Ser Gly Lys Lys Gln Lys Lys
        195                 200                 205

Lys Thr Leu Ala Glu Ile Asn Gln Lys Trp Asn Leu Glu Ala Glu Lys
    210                 215                 220

Glu Asp Gly Glu Phe Asp Ser Lys Glu Glu Ser Lys Gln Lys Leu Val
225                 230                 235                 240

Ser Phe Cys Ser Gln Pro Ser Val Ile Ser Ser Pro Gln Ile Asn Gly
                245                 250                 255

Glu Ile Asp Leu Leu Ala Ser Gly Ser Leu Thr Glu Ser Glu Cys Phe
            260                 265                 270

Gly Ser Leu Thr Glu Val Ser Leu Pro Leu Ala Glu Gln Ile Glu Ser
        275                 280                 285

Pro Asp Thr Lys Ser Arg Asn Glu Val Val Thr Pro Glu Lys Val Cys
    290                 295                 300

Lys Asn Tyr Leu Thr Ser Lys Lys Ser Leu Pro Leu Glu Asn Asn Gly
305                 310                 315                 320

Lys Arg Gly His His Asn Arg Leu Ser Ser Pro Ile Ser Lys Arg Cys
                325                 330                 335

Arg Thr Ser Ile Leu Ser Thr Ser Gly Asp Phe Val Lys Gln Thr Val
            340                 345                 350

Pro Ser Glu Asn Ile Pro Leu Pro Glu Cys Ser Ser Pro Pro Ser Cys
        355                 360                 365

Lys Arg Lys Val Gly Gly Thr Ser Gly Arg Lys Asn Ser Asn Met Ser
    370                 375                 380

Asp Glu Phe Ile Ser Leu Ser Pro Gly Thr Pro Pro Ser Thr Leu Ser
385                 390                 395                 400

Ser Ser Ser Tyr Arg Gln Val Met Ser Ser Pro Ser Ala Met Lys Leu
                405                 410                 415

Leu Pro Asn Met Ala Val Lys Arg Asn His Arg Gly Glu Thr Leu Leu
            420                 425                 430

His Ile Ala Ser Ile Lys Gly Asp Ile Pro Ser Val Glu Tyr Leu Leu
        435                 440                 445

Gln Asn Gly Ser Asp Pro Asn Val Lys Asp His Ala Gly Trp Thr Pro

6

450                 455                 460

Leu His Glu Ala Cys Asn His Gly His Leu Lys Val Val Glu Leu Leu
465                 470                 475                 480

Leu Gln His Lys Ala Leu Val Asn Thr Thr Gly Tyr Gln Asn Asp Ser
                485                 490                 495

Pro Leu His Asp Ala Ala Lys Asn Gly His Val Asp Ile Val Lys Leu
            500                 505                 510

Leu Leu Ser Tyr Gly Ala Ser Arg Asn Ala Val Asn Ile Phe Gly Leu
        515                 520                 525

Arg Pro Val Asp Tyr Thr Asp Asp Glu Ser Met Lys Ser Leu Leu Leu
    530                 535                 540

Leu Pro Glu Lys Asn Glu Ser Ser Ser Ala Ser His Cys Ser Val Met
545                 550                 555                 560

Asn Thr Gly Gln Arg Arg Asp Gly Pro Leu Val Leu Ile Gly Ser Gly
                565                 570                 575

Leu Ser Ser Glu Gln Gln Lys Met Leu Ser Glu Leu Ala Val Ile Leu
            580                 585                 590

Lys Ala Lys Lys Tyr Thr Glu Phe Asp Ser Thr Val Thr His Val Val
        595                 600                 605

Val Pro Gly Asp Ala Val Gln Ser Thr Leu Lys Cys Met Leu Gly Ile
    610                 615                 620

Leu Asn Gly Cys Trp Ile Leu Lys Phe Glu Trp Val Lys Ala Cys Leu
625                 630                 635                 640

Arg Arg Lys Val Cys Glu Gln Glu Glu Lys Tyr Glu Ile Pro Glu Gly
                645                 650                 655

Pro Arg Arg Ser Arg Leu Asn Arg Glu Gln Leu Leu Pro Lys Leu Phe
            660                 665                 670

Asp Gly Cys Tyr Phe Tyr Leu Trp Gly Thr Phe Lys His His Pro Lys
        675                 680                 685

Asp Asn Leu Ile Lys Leu Val Thr Ala Gly Gly Gly Gln Ile Leu Ser
    690                 695                 700

Arg Lys Pro Lys Pro Asp Ser Asp Val Thr Gln Thr Ile Asn Thr Val
705                 710                 715                 720

Ala Tyr His Ala Arg Pro Asp Ser Asp Gln Arg Phe Cys Thr Gln Tyr
                725                 730                 735

Ile Ile Tyr Glu Asp Leu Cys Asn Tyr His Pro Glu Arg Val Arg Gln
            740                 745                 750

Gly Lys Val Trp Lys Ala Pro Ser Ser Trp Phe Ile Asp Cys Val Met
        755                 760                 765

Ser Phe Glu Leu Leu Pro Leu Asp Ser
    770                 775